(19) Europäisches Patentamt / European Patent Office / Office européen des brevets

(11) **EP 3 221 470 B1**

(12) **EUROPEAN PATENT SPECIFICATION**

(45) Date of publication and mention of the grant of the patent:
**14.08.2019 Bulletin 2019/33**

(21) Application number: **15820291.1**

(22) Date of filing: **17.11.2015**

(51) Int Cl.:
*C12Q 1/689* (2018.01)     *G01N 33/50* (2006.01)
*C12Q 1/10* (2006.01)

(86) International application number:
**PCT/IL2015/051102**

(87) International publication number:
**WO 2016/079731 (26.05.2016 Gazette 2016/21)**

(54) **METHOD OF ANALYZING MICROBIOME**

VERFAHREN ZUR ANALYSE EINES MIKROBIOMS

PROCÉDÉ D'ANALYSE DE MICROBIOME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.11.2014 US 201462080466 P**

(43) Date of publication of application:
**27.09.2017 Bulletin 2017/39**

(73) Proprietor: **Yeda Research and Development Co., Ltd.**
**7610002 Rehovot (IL)**

(72) Inventors:
• **SEGAL, Eran**
  **4730168 Ramat-HaSharon (IL)**
• **ELINAV, Eran**
  **7680400 Mazkeret Batia (IL)**

(74) Representative: **Dennemeyer & Associates S.A.**
**Postfach 70 04 25**
**81304 München (DE)**

(56) References cited:
• **C. A. MULLER ET AL: "The dynamics of genome replication using deep sequencing", NUCLEIC ACIDS RESEARCH, vol. 42, no. 1, 1 October 2013 (2013-10-01), pages e3-e3, XP055250078, GB ISSN: 0305-1048, DOI: 10.1093/nar/gkt878**
• **ERIK A. PELVE ET AL: "Mapping of active replication origins in vivo in thaum- and euryarchaeal replicons", MOLECULAR MICROBIOLOGY., vol. 90, no. 3, 16 November 2013 (2013-11-16), pages 538-550, XP055250345, GB ISSN: 0950-382X, DOI: 10.1111/mmi.12382**
• **JIA XU ET AL: "Genome-wide identification and characterization of replication origins by deep sequencing", GENOME BIOLOGY, BIOMED CENTRAL LTD., LONDON, GB, vol. 13, no. 4, 24 April 2012 (2012-04-24) , page R27, XP021096290, ISSN: 1465-6906, DOI: 10.1186/GB-2012-13-4-R27**
• **SCHMIDT ET AL: "Comparison of growth phase on Salmonella enterica serovar Typhimurium invasion in an epithelial cell line (IPEC J2) and mucosal explants from porcine small intestine", COMPARATIVE IMMUNOLOGY, MICROBIOLOGY AND INFECTIOUS DISEASES, PERGAMON PRESS, OXFORD, GB, vol. 31, no. 1, 21 November 2007 (2007-11-21), pages 63-69, XP022355852, ISSN: 0147-9571, DOI: 10.1016/J.CIMID.2007.04.003**
• **Santosh Dulal ET AL: "Gut Microbiome and Colorectal Adenomas :", CANCER JOURNAL, vol. 20, no. 3, 1 January 2014 (2014-01-01), pages 225-231, XP055462930, US ISSN: 1528-9117, DOI: 10.1097/PPO.0000000000000050**
• **T. Korem ET AL: "Growth dynamics of gut microbiota in health and disease inferred from single metagenomic samples", SCIENCE, vol. 349, no. 6252, 4 September 2015 (2015-09-04), pages 1101-1106, XP55557436, US ISSN: 0036-8075, DOI: 10.1126/science.aac4812**

**Description**

FIELD AND BACKGROUND OF THE INVENTION

The present invention, in some embodiments thereof, relates to a method of analyzing metagenomic data of the microbiome so as to obtain information regarding growth dynamics of the microbes therein.

The human in intestine carries a vast and diverse microbial ecosystem that has co-evolved with our species and is essential for human health. Mammals possess an 'extended genome' of millions of microbial genes located in the intestine: the microbiome. This multigenomic symbiosis is expressed at the proteomic and metabolic levels in the host and it has therefore been proposed that humans represent a vastly complex biological 'superorganism' in which part of the responsibility for host metabolic regulation is devolved to the microbial symbionts. Modern interpretation of the gut microbiome is based on a culture-independent, molecular view of the intestine provided by high-throughput genomic screening technologies. Also, the gut microbiome has been directly implicated in the etiopathogenesis of a number of pathological states as diverse as obesity, circulatory disease, inflammatory bowel diseases (IBDs) and autism. The gut microbiota also influences drug metabolism and toxicity, dietary calorific bioavailability, immune system conditioning and response, and post-surgical recovery. The implication is that quantitative analysis of the gut microbiome and its activities is essential for the generation of future personalized healthcare strategies and that the gut microbiome represents a fertile ground for the development of the next generation of therapeutic drug targets. It also implies that the gut microbiome may be directly modulated for the benefit of the host organism.

Traditionally, studying microbial samples from human skin, stool, or blood relied on time- and labor-intensive microbiology techniques of growing and isolating individual organisms followed by phenotypic or genotypic analysis. Microbial community profiling within a single sample was not possible with these methods.

The advent of next-generation sequencing (NGS) enabled several high-profile collaborative projects including the Human Microbiome Project and MetaHIT, which have published a wide range of data on the human microbiome using NGS as a foundational tool.

Research into the microbiome typically attempts to characterize the microbiome by identifying the microbes contained within and the genetic make-up thereof.

Background art includes Xu, J. et al. Genome Biol. 13, R27 (2012) and Skovgaard, O., et al Genome Res. 21, 1388-93 (2011).

SUMMARY OF THE INVENTION

According to an aspect of some embodiments of the present invention there is provided a method of assessing the growth dynamics of a bacterium in a microbiome comprising: (a) sequencing DNA fragments of a microbiome to obtain a plurality of nucleic acid sequencing data; (b) aligning the plurality of nucleic acid sequence data to at least one reference sequence, the reference sequence being of a genome of the bacterium; and (c) analyzing the frequency of at least one nucleotide positioned at the origin of replication of the genome and the frequency of at least one nucleotide positioned at the terminus of the genome, wherein the ratio of the frequencies is indicative of the growth dynamics of the bacterium.

According to an aspect of some embodiments of the present disclosure there is provided a method of determining the origin of replication of a replicating bacterium comprising:

(a) sequencing DNA fragments of the genome of the bacterium to obtain a plurality of nucleic acid sequencing data;
(b) aligning the plurality of nucleic acid sequence data to a reference sequence, the reference sequence being of the genome of the bacterium; and
(c) analyzing the frequency of nucleotides across the genome of the bacterium; wherein the genomic location which corresponds to the highest number of reads is the origin of replication of the bacterium.

According to an aspect of some embodiments of the present disclosure there is provided a method of analyzing the health of a test microbiome comprising:

(a) analyzing the growth dynamics of at least one bacterium in the test microbiome;
(b) comparing the growth dynamics of the at least one bacterium in the test microbiome to the growth dynamics of the at least one bacterium in a pathological microbiome, wherein when the growth dynamics of the at least one bacterium in the test microbiome is statistically significantly similar to the growth dynamics of the at least one bacterium in the pathological microbiome, it is indicative that the microbiome is not healthy.

According to an aspect of some embodiments of the present invention there is provided a method of determining the health of a subject comprising:

(a) analyzing the growth dynamics of at least one bacteria in a microbiome sample of the subject;
(b) comparing the growth dynamics of the at least one bacteria in the microbiome sample of the subject to the growth dynamics of the at least one bacteria in at least one pathological microbiome, wherein when the growth dynamics of the at least one bacteria in the microbiome sample is statistically significantly similar to the growth dynamics of the at least one bacteria in the pathological microbiome, it is indicative that the subject is not healthy.

According to some embodiments of the invention, the method further comprises fragmenting DNA of the microbiome to obtain the DNA fragments of the microbiome.

According to some embodiments of the disclosure step (c) comprises analyzing the frequency of each of the nucleotide across the genome of the bacterium.

According to some embodiments of the invention, the sequencing comprises parallel high throughput sequencing.

According to some embodiments of the invention, the high throughput sequencing comprises deep sequencing.

According to some embodiments of the disclosure, the method further comprises graphically displaying the frequency of the nucleotides as a function of its genomic location.

According to some embodiments of the invention, the microbiome is selected from the group consisting of a skin microbiome, a gut microbiome, an intestinal microbiome, a mouth microbiome and a vaginal microbiome.

According to some embodiments of the invention, the microbiome is a human microbiome.

According to some embodiments of the invention, the microbiome comprises a gut microbiome.

According to some embodiments of the invention, when the frequency of the nucleotide at the origin of replication of the genome : frequency of the nucleotide at the terminus of the genome is about 2:1 or more, it is indicative of exponential growth of the bacterium.

According to some embodiments of the invention, when the frequency of a nucleotide at the origin of replication of the genome : frequency of a nucleotide at the terminus of the genome is about 1:1, it is indicative of stationary growth of the bacterium.

According to some embodiments of the invention, the method further comprises fragmenting DNA of the microbiome to obtain the DNA fragments of the microbiome.

According to some embodiments of the disclosure, the method further comprises graphically displaying the frequency of the nucleotides as a function of its genomic location.

According to some embodiments of the invention, the sequencing comprises high throughput sequencing.

According to some embodiments of the invention, the high throughput sequencing comprises deep sequencing.

According to some embodiments of the invention, the bacterium is comprised in a mixed population of bacteria.

According to some embodiments of the invention, the mixed population of bacteria comprises a microbiome.

According to some embodiments of the invention, the microbiome comprises a gut microbiome.

According to some embodiments of the invention, the analyzing the growth dynamics is effected according to the method described herein.

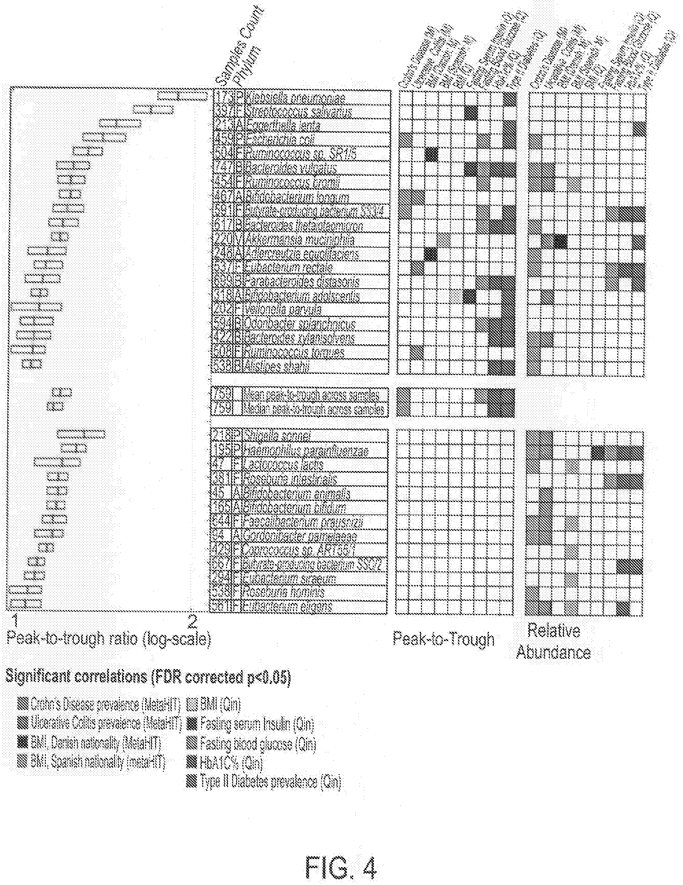

According to some embodiments of the invention, the bacteria is selected from the group consisting of those set forth in Figure 4.

According to some embodiments of the invention, the pathological microbiome is derived from the gut of a subject with a metabolic disorder.

According to some embodiments of the invention, the metabolic disorder is selected from the group consisting of Diabetes, obesity, ulcerative colitis and Crohn's disease.

According to some embodiments of the invention, the test microbiome and the pathological microbiome are gut microbiomes.

According to some embodiments of the invention, the analyzing the growth dynamics is effected according to the method described herein.

According to some embodiments of the invention, the at least one bacteria is selected from the group consisting of those set forth in Figure 4.

According to some embodiments of the invention, the microbiome sample comprises a gut microbiome sample.

According to some embodiments of the invention, the pathological microbiome is derived from the gut of a subject with a metabolic disorder.

According to some embodiments of the invention, the metabolic disorder is selected from the group consisting of Diabetes, obesity, ulcerative colitis and Crohn's disease.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting. The invention is defined in the appended claims.

BRIEF DESCRIPTION OF THE SEVERAL VIEWS OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

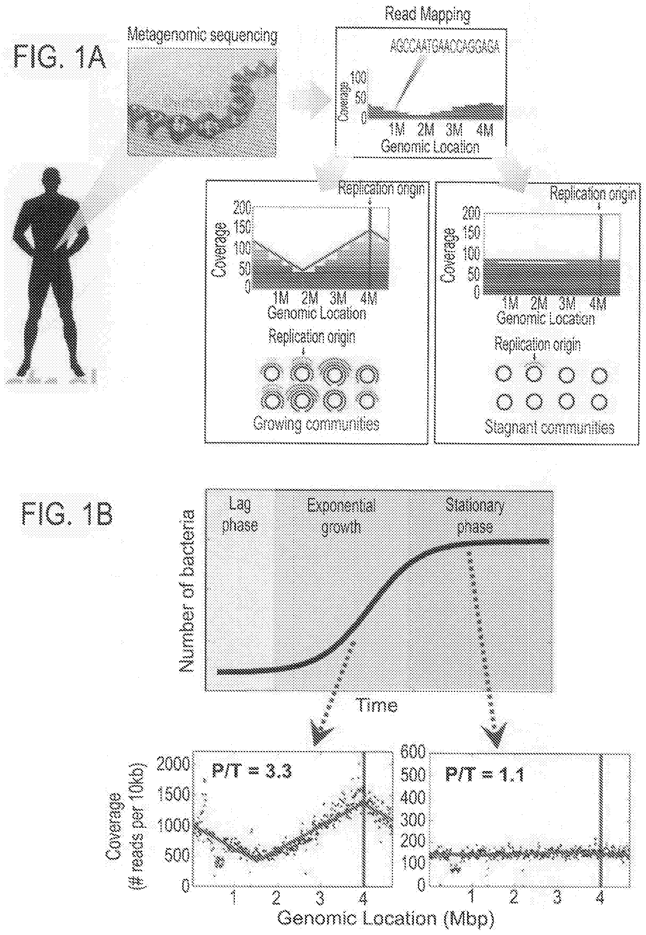

FIGs. 1A-C. A signature of *E. coli* growth dynamics obtained from a single metagenomic sample. (A) Illustration of the present approach for inferring bacterial growth dynamics from sequencing coverage analysis of a single metagenomic sample. Sequencing reads from a metagenomic sample are mapped to available bacterial genomes and the sequencing coverage (number of mapped metagenomic reads) at each genomic location is then plotted across the entire length of every bacterial genome (graph). Individual bacteria of a growing bacterial population (top) will each be found at a different stage of DNA replication, generating a coverage pattern that peaks near the known replication origin (green vertical line in graph), and producing a prototypical sequencing coverage pattern with a single peak and a single trough. Bacteria from a non-dividing population (bottom) each have a single copy of the genome, producing a flat sequencing coverage pattern across the genome. (B) Sequencing coverage of an isolated *E. coli* culture grown ex-vivo and extracted during the exponential growth phase (left) or the stationary phase (right). Dots represent the number of reads at each genomic location in non-overlapping 10Kbp windows. Blue lines represent linear piecewise fits to the dots that reside between the peak and trough locations. Peak-to-trough ratios are indicated, computed by dividing the sequencing coverage at the replication origin by that at the trough (Methods). (C) Sequencing coverage of *E. coli* across 522 different human gut metagenomic samples[7,9]. Shown is a histogram of the number of metagenomic samples (y-axis) as a function of the peak-to-trough ratio (x-axis), and three examples of sequencing coverage patterns plotted as in (B) and taken from different ranges of peak-to-trough ratios of the histogram. P/T - peak-to-trough ratio.

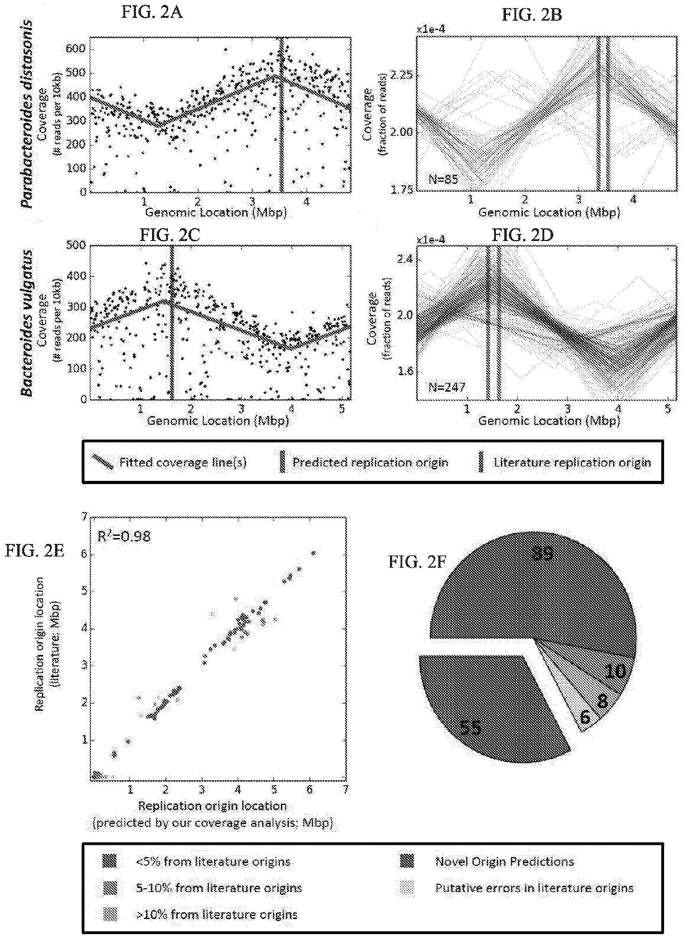

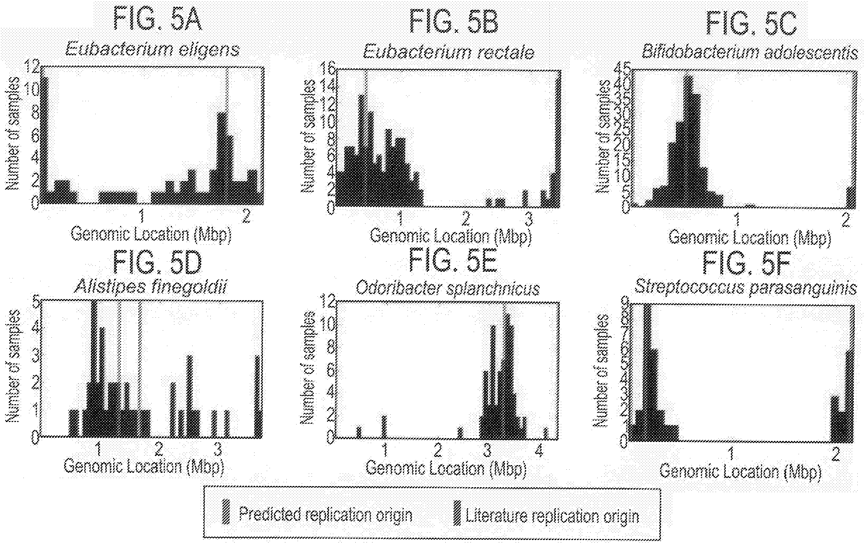

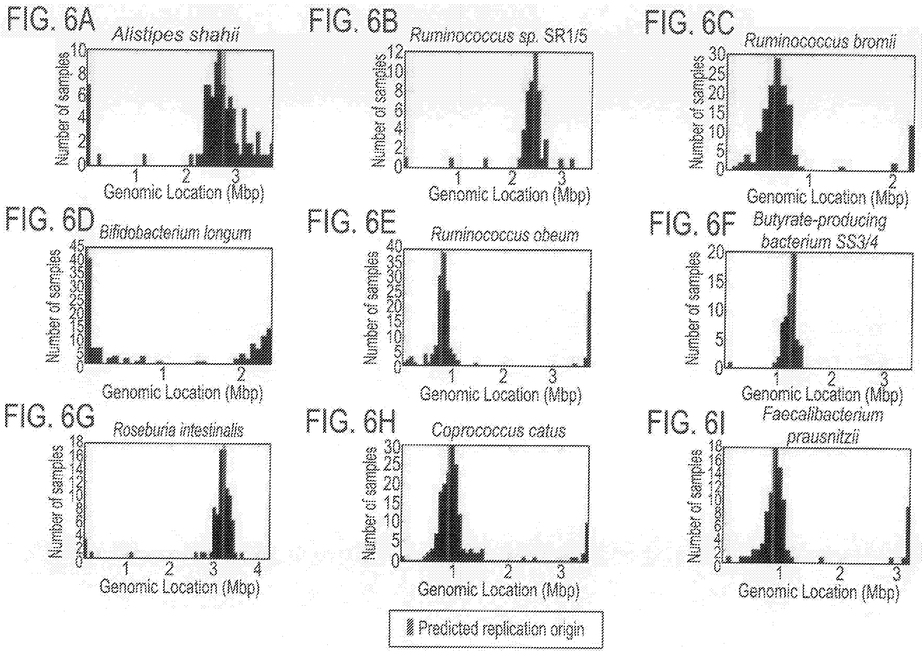

FIGs. 2A-F. Coverage analysis accurately identifies origins of replication. (A) Coverage analysis of *Parabacteroides distasonis* from one metagenomic sample. (B) Sequencing coverage fitted lines (blue) of *P. distasonis* obtained from 85 different human gut metagenomic samples. The known replication origin location is shown (green vertical line) together with the location of the origin predicted from our coverage analysis by taking the circular-median of fitted peaks across all samples (red vertical line; Methods). (C,D) Same as (A,B) but for *Bacteroides vulgatus.* (E) Replication origin locations obtained from the literature[30] (y-axis) or predicted by our sequencing coverage analysis (x-axis) for 113 bacteria that have a reported origin out of the 168 bacteria for which we identified peak-to-trough prototypical patterns (Methods) across 346 publicly available metagenomic stool samples[9]. Each dot represents one bacteria and is colored according to the distance between its literature location and its predicted location (measured in the fraction of the bacterial genome, see legend). Pearson correlation ($R^2$) is shown. (F) Pie chart indicating the number of bacteria for different categories of the match between the known and predicted replication origin location (three different shades of green for distances <5%, 5-10%, or >10% of the bacterial genome length); for replication origins whose location is likely misplaced (Figures 5A-F), and for novel replication origins predicted by our coverage analysis that have no known origin (Figures 6A-I).

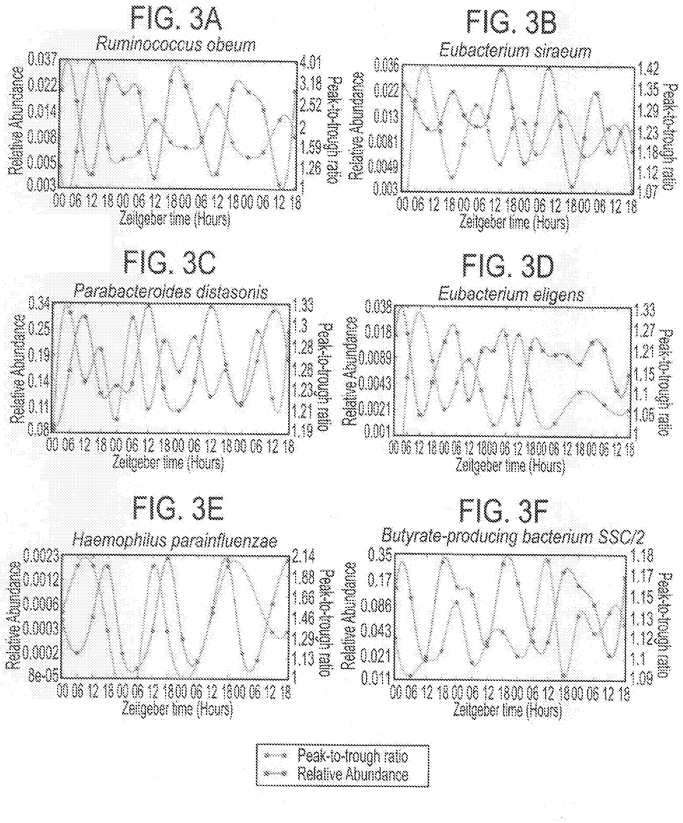

FIGs. 3A-F. Bacterial dynamics exhibit diurnal oscillations that are out of phase with oscillations in abundance levels. (A) Relative abundance levels (red) and peak-to-trough ratios (blue) of *Ruminococcus obeum* across metagenomic samples obtained approximately every 6 hours from one human individual in 4 consecutive days. Peak-to-trough ratios were computed by the coverage analysis method (Methods) and are shown in log-scale. Time is represented by Zeitgeber time (ZT, x-axis). Note that peak-to-trough ratios rise to their highest value 6 hours before the rise in relative abundance levels. (B-D) Same as (A), for Eubacterium siraeum (B), *Parabacteroides distasonis* (C), and *Haemophilus parainfluenzae* (D).

FIG. 4. Bacterial dynamics correlate with several diseases and metabolic disorders. Peak to trough (PTR) ratios of species from Chinese (N=363; Q) and European (N=396;M) cohorts are shown (boxplots, left; red-median boundaries 25-75 percentiles) if its relative abundances or PTRs were significantly associated with clinical parameters. Shown are phylum membership; the number of samples for which PTRs were calculated; and a row with colored entries for each statistically significant (FDR-corrected P is less than 0.5) association between clinical parameters and its PTR (left column block) or relative abundance (right column block). Mann-Whitney U-test and Spearman correlations were used for binary and continuous clinical parameters respectively. Top-block: species with significant associates between PTR and clinical parameters; bottom block: species with significant association only between relative abundance and clinical parameters.

The phylum of each bacteria is indicated (A-Actinobacteria, B-Bacteroidetes, F-Firmicutes, P-Proteobacteria, V-Verrucomicrobia).

FIGs. 5A-F. Sequencing coverage analysis identifies misplaced replication origin locations. Shown are histograms of the number of samples with fitted peak location (y-axis) as a function of the genomic location in bins of 70Kbp (x-axis) for 6 bacteria in which the analysis suggests that the known replication origins are misplaced. The known (green) and predicted (red) replication origin location are shown as in Figures 2A-F. Note that in all cases shown, peak locations agree well with each other across different human gut metagenomic samples but not with the replication origin location reported in the literature, suggesting that the latter is misplaced.

FIGs. 6A-I. Sequencing coverage analysis identifies novel replication origin locations. Shown are histograms as in Figures 5A-F, for 9 out of 55 bacteria in which the analysis predicts a replication origin location but no such origin is known in the literature. Note that in all cases shown, peak locations agree well with each other across different human gut metagenomic samples, suggesting that they indeed represent novel replication origins.

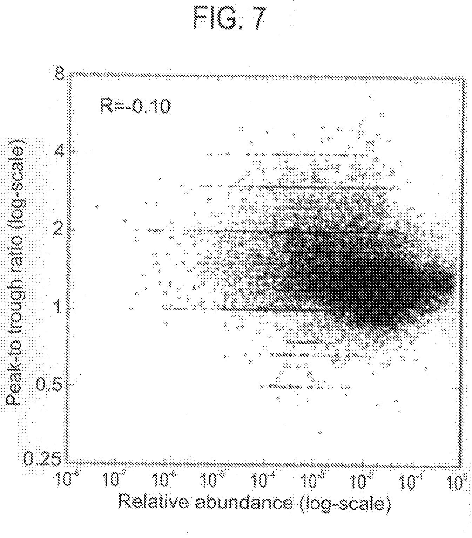

FIG. 7. Bacteria growth dynamics are independent of relative abundance levels. Shown is a dot plot in which each dot represents the peak-to-trough ratio (y-axis) and relative abundance (x-axis) of one bacteria in a single metagenomic sample. All 207 bacteria for which coverage analysis was performed are shown across all 709 metagenomic samples analyzed. Note that there is relatively little correlation between peak-to-trough ratios and relative abundance levels, indicating that peak-to-trough ratios provide information that is largely independent of that provided by bacterial relative abundances.

## DESCRIPTION OF SPECIFIC EMBODIMENTS OF THE INVENTION

The present invention, in some embodiments thereof, relates to a method of analyzing metagenomic data of the microbiome so as to obtain information regarding growth dynamics of the microbes therein.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways. The scope of the invention is defined in the appended claims.

Characterization of microbiome composition and function through shotgun sequencing has provided many insights into its complex roles in health and disease. This was accomplished using several analysis techniques, including gene calling, functional/pathway analysis, metagenomic-wide association studies, genome assembly, and metagenomic single nucleotide polymorphism (SNPs) detection. These approaches were highly valuable in establishing associations between microbiome configurations and susceptibility to several diseases, including obesity, adult-onset diabetes mellitus, auto-inflammatory disorders, metabolic disease and even cancer. However, a fundamental limitation of these approaches is that they treat the microbiota ecosystem as a static snapshot at the point of collection, disregarding its highly dynamic nature and the differential activity state of its many microbial members.

The present inventors have now asked whether microbiota dynamics could be probed from a single metagenomic sample by examining the pattern of sequencing read coverage (depth) across bacterial genomes, i.e., the variation in the number of metagenomic reads mapped to different genomic regions. Most bacteria harbor a single circular chromosome which replicates bi-directionally from a single fixed origin towards a single terminus. Thus, during bacterial DNA replication, regions already passed by the replication fork will have two copies compared to the single copy of the yet unreplicated regions. This idea also holds in an asynchronous bacterial population in which every cell may be at a different stage of replication, since summed across the population, the copy number of a DNA region should be higher the closer that region is to the replication origin, and lower the closer that region is to the terminus. Moreover, since DNA replication rate is generally constant, the quantitative ratio between the DNA copy number near the replication origin and that near the terminus should reflect the growth rate of the bacterial population. This is because in a faster growing population featuring shorter generation times, more microbial cells are engaged in DNA replication and thus more active replication points will be present in each cell, resulting in a higher than 1:1 ratio between near-origin DNA and near-terminus DNA. When generation time is shorter than DNA replication time, this origin to terminus ratio may even be greater than 2:1 due to multifork replication.

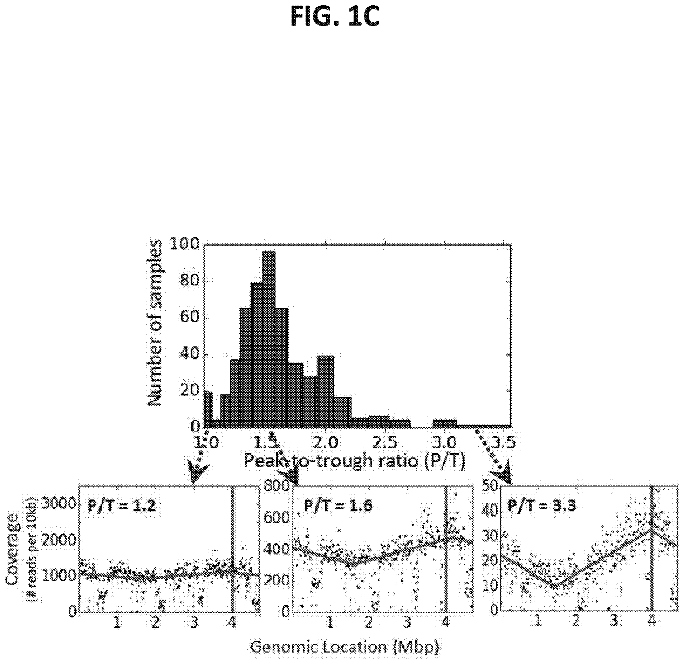

Notably, by separately applying coverage analysis to 709 mixed asynchronous microbiome populations from human gut metagenomic samples, the present inventors unraveled a clear prototypical pattern that persisted across different bacteria and samples, comprising of a single coverage peak and a single trough, with the distance between them being roughly half the length of the bacterial genome (Figures 1C and 2A-D). Locations of known replication origins were in excellent agreement with the peaks of sequencing coverage (113 bacteria), whereas for 55 other bacteria whose replication origin is unknown, novel origins with a robust coverage peak across multiple samples were predicted (Figures 2E-F). Peak-to-trough coverage ratios vary greatly across gut metagenomic samples of different human individuals, with high and low ratios being similar to those obtained in exponentially growing and stationary phase bacterial populations grown in culture ex-vivo, respectively. Within the same individual, ratios of some bacteria also exhibit diurnal oscillations that are out of phase with the oscillations in the abundance levels of these same bacteria, together suggesting that these ratios are indeed reflective of bacterial growth dynamics (Figures 3A-F). Finally, peak-to-trough ratios exhibit significant correlations with several host parameters, including HbA1C%, fasting glucose levels, and prevalence of adult-onset diabetes mellitus and Crohn's disease (Figure 4).

Thus, overall, by analyzing patterns of bacterial sequence coverage, the present examples provide novel insights into dynamics of bacterial growth from a single metagenomic sample, which may bear clinical relevance.

Thus, according to one aspect of the present invention there is provided a method of assessing the growth rate of a bacterium in a microbiome comprising:

(a) sequencing DNA fragments of a microbiome to obtain a plurality of nucleic acid sequencing data;
(b) aligning the plurality of nucleic acid sequence data to at least one reference sequence, the reference sequence being of a genome of the bacterium; and
(c) analyzing the frequency of at least one nucleotide positioned at the origin of replication of the genome and the frequency of at least one nucleotide positioned at the terminus of the genome, wherein the ratio of the frequencies is indicative of the growth dynamics of the bacterium.

The term "growth dynamics" refers to the growth phase of a bacterium (e.g. lag phase, stationary phase, exponential growth, death phase) and to the growth rate itself.

During lag phase, bacteria adapt themselves to growth conditions. It is the period where the individual bacteria are maturing and not yet able to divide. During the lag phase of the bacterial growth cycle, synthesis of RNA, enzymes and other molecules occurs.

The log phase (sometimes called the logarithmic phase or the *exponential phase)* is a period characterized by cell doubling. The number of new bacteria appearing per unit time is proportional to the present population. If growth is not limited, doubling will continue at a constant rate so both the number of cells and the rate of population increase doubles with each consecutive time period. For this type of exponential growth, plotting the natural logarithm of cell number against time produces a straight line. The slope of this line is the specific growth rate of the organism, which is a measure of the number of divisions per cell per unit time. The actual rate of this growth depends upon the growth conditions, which affect the frequency of cell division events and the probability of both daughter cells surviving. Exponential growth cannot continue indefinitely, however, because the medium is soon depleted of nutrients and enriched with wastes.

The stationary phase is often due to a growth-limiting factor such as the depletion of an essential nutrient, and/or the formation of an inhibitory product such as an organic acid. Stationary phase results from a situation in which growth rate and death rate are equal. The number of new cells created is limited by the growth factor and as a result the rate of cell growth matches the rate of cell death.

At death phase, (Decline phase) bacteria die. This could be due to lack of nutrients, a temperature which is too high or low, or the wrong living conditions.

As used herein, the term "microbiome" refers to the totality of microbes (bacteria, fungae, protists), their genetic elements (genomes) in a defined environment.

The microbiome may be of any origin - for example a gut microbiome, an oral microbiome, an intestinal microbiome, a bronchial microbiome, a skin microbiome or a vaginal microbiome.

According to a particular embodiment, the microbiome is a gut microbiome.

In order to analyze the microbiome, samples are taken from a subject.

The subject is typically a mammalian subject - e.g. human subject.

Thus, for example stool samples may be taken to analyze the gut microbiome, bronchial samples may be taken to analyze the bronchial microbiome, a saliva sample may be taken to analyze the oral microbiome etc. According to a particular embodiment, the microbiome of a subject is derived from a stool sample of the subject.

The present inventors have shown that changes in eating patterns (e.g. due to circadian misalignment) affect the composition of the microbiome. Therefore, preferably samples are taken at a fixed time in the day.

The bacterium whose growth dynamics is being analyzed may be a gram positive or gram negative bacterium.

The term "Gram-positive bacteria" as used herein refers to bacteria characterized by having as part of their cell wall structure peptidoglycan as well as polysaccharides and/or teichoic acids and are characterized by their blue-violet color reaction in the Gram-staining procedure. Representative Gram-positive bacteria include: Actinomyces spp., Bacillus anthracis, Bifidobacterium spp., Clostridium botulinum, Clostridium perfringens, Clostridium spp., Clostridium tetani, Corynebacterium diphtheriae, Corynebacterium jeikeium, Enterococcus faecalis, Enterococcus faecium, Erysipelothrix rhusiopathiae, Eubacterium spp., Gardnerella vaginalis, Gemella morbillorum, Leuconostoc spp., Mycobacterium abcessus, Mycobacterium avium complex, Mycobacterium chelonae, Mycobacterium fortuitum, Mycobacterium haemophilium, Mycobacterium kansasii, Mycobacterium leprae, Mycobacterium marinum, Mycobacterium scrofulaceum, Mycobacterium smegmatis, Mycobacterium terrae, Mycobacterium tuberculosis, Mycobacterium ulcerans, Nocardia spp., Peptococcus niger, Peptostreptococcus spp., Proprionibacterium spp., Staphylococcus aureus, Staphylococcus auricularis, Staphylococcus capitis, Staphylococcus cohnii, Staphylococcus epidermidis, Staphylococcus haemolyticus, Staphylococcus hominis, Staphylococcus lugdanensis, Staphylococcus saccharolyticus, Staphylococcus saprophyticus, Staphylococcus schleiferi, Staphylococcus similans, Staphylococcus warneri, Staphylococcus xylosus, Streptococcus agalactiae (group B streptococcus), Streptococcus anginosus, Streptococcus bovis, Streptococcus canis, Streptococcus equi, Streptococcus milleri, Streptococcus mitior, Streptococcus mutans, Streptococcus pneumoniae, Streptococcus pyogenes (group A streptococcus), Streptococcus salivarius, Streptococcus sanguis.

The term "Gram-negative bacteria" as used herein refer to bacteria characterized by the presence of a double membrane surrounding each bacterial cell. Representative Gram-negative bacteria include Acinetobacter calcoaceticus, Actinobacillus actinomycetemcomitans, Aeromonas hydrophila, Alcaligenes xylosoxidans, Bacteroides, Bacteroides fragilis, Bartonella bacilliformis, Bordetella spp., Borrelia burgdorferi, Branhamella catarrhalis, Brucella spp., Campylobacter spp., Chalmydia pneumoniae, Chlamydia psittaci, Chlamydia trachomatis, Chromobacterium violaceum, Citrobacter spp., Eikenella corrodens, Enterobacter aerogenes, Escherichia coli, Flavobacterium meningosepticum, Fusobacterium spp., Haemophilus influenzae, Haemophilus spp., Helicobacter pylori, Klebsiella spp., Legionella spp., Leptospira spp., Moraxella catarrhalis, Morganella morganii, Mycoplasma pneumoniae, Neisseria gonorrhoeae, Neisseria meningitidis, Pasteurella multocida, Plesiomonas shigelloides, Prevotella spp., Proteus spp., Providencia rettgeri, Pseudomonas aeruginosa, Pseudomonas spp., Rickettsia prowazekii, Rickettsia rickettsii, Rochalimaea spp., Salmonella spp., Salmonella typhi, Serratia marcescens, Shigella spp., Treponema carateum, Treponema pallidum, Treponema pallidum endemicum, Treponema pertenue, Veillonella spp., Vibrio cholerae, Vibrio vulnificus, Yersinia enterocolitica, Yersinia pestis.

Typically the genome sequence of the bacterium whose growth dynamics is being analyzed is known (or at least a sub-portion thereof is known). The known sequence is referred to herein as the reference sequence and is further described herein below.

Obtaining chromosomal (genomic) DNA from microbiomes may be effected using conventional techniques, for example as disclosed in Sambrook and Russell, Molecular Cloning: A Laboratory Manual, cited supra. In some cases, particularly if small amounts of DNA are employed in a particular step, it is advantageous to provide carrier DNA, e.g. unrelated circular synthetic double-stranded DNA, to be mixed and used with the sample DNA whenever only small amounts of sample DNA are available and there is danger of losses through nonspecific binding, e.g. to container walls and the like.

In one embodiment, long fragments of chromosomal DNA are obtained. Cells are lysed and the intact nuclei may be pelleted with a gentle centrifugation step. The genomic DNA is then released (e.g. through proteinase K and RNase digestion, for several hours (e.g. 1-5 hours)). The material can be treated to lower the concentration of remaining cellular waste, e.g., by dialysis for a period of time (i.e., from 2-16 hours) and/or dilution. Since such methods need not employ many disruptive processes (such as ethanol precipitation, centrifugation, and vortexing), the genomic nucleic acid remains largely intact, yielding a majority of fragments that have lengths in excess of 150 kilobases. In some embodiments, the fragments are from about 5 to about 750 kilobases in lengths. In further embodiments, the fragments are from about 150 to about 600, about 200 to about 500, about 250 to about 400, and about 300 to about 350 kilobases in length.

Optionally, the target genomic DNA is then fractionated or fragmented to a desired size by conventional techniques including enzymatic digestion, shearing, or sonication, with the latter two finding particular use in the present invention.

Fragment sizes of the target nucleic acid can vary depending on the source target nucleic acid, and the library construction methods used, but for standard whole-genome sequencing such fragments may range from 50 to 600 nucleotides in length. In another embodiment, the fragments are 300 to 600 or 200 to 2000 nucleotides in length. In yet another embodiment, the fragments are 10-100, 50-100, 50-300, 100-200, 200-300, 50-400, 100-400, 200-400, 300-400, 400-500, 400-600, 500-600, 50-1000, 100-1000, 200-1000, 300-1000, 400-1000, 500-1000, 600-1000, 700-1000, 700-900, 700-800, 800-1000, 900-1000, 1500-2000. 1750-2000, and 50-2000 nucleotides in length. Longer fragments are also contemplated.

In a further embodiment, fragments of a particular size or in a particular range of sizes are isolated. Such methods are well known in the art. For example, gel fractionation can be used to produce a population of fragments of a particular size within a range of basepairs, for example for 500 base pairs+50 base pairs.

In many cases, enzymatic digestion of extracted DNA is not required because shear forces created during lysis and extraction will generate fragments in the desired range. In a further embodiment, shorter fragments (1-5 kb) can be generated by enzymatic fragmentation using restriction endonucleases.

Methods for sequence determination are generally known to the person skilled in the art. Preferred sequencing methods are next generation sequencing methods or parallel high throughput sequencing methods. For example, a bacterial genomic sequence may be obtained by using Massively Parallel Signature Sequencing (MPSS). An example of an envisaged sequence method is pyrosequencing, in particular 454 pyrosequencing, e.g. based on the Roche 454 Genome Sequencer. This method amplifies DNA inside water droplets in an oil solution with each droplet containing a single DNA template attached to a single primer-coated bead that then forms a clonal colony. Pyrosequencing uses luciferase to generate light for detection of the individual nucleotides added to the nascent DNA, and the combined data are used to generate sequence read-outs. Yet another envisaged example is Illumina or Solexa sequencing, e.g. by using the Illumina Genome Analyzer technology, which is based on reversible dye-terminators. DNA molecules are typically attached to primers on a slide and amplified so that local clonal colonies are formed. Subsequently one type of nucleotide at a time may be added, and non-incorporated nucleotides are washed away. Subsequently, images of the fluorescently labeled nucleotides may be taken and the dye is chemically removed from the DNA, allowing a next cycle. Yet another example is the use of Applied Biosystems' SOLiD technology, which employs sequencing by ligation. This method is based on the use of a pool of all possible oligonucleotides of a fixed length, which are labeled according to the sequenced position. Such oligonucleotides are annealed and ligated. Subsequently, the preferential ligation by DNA ligase for matching sequences typically results in a signal informative of the nucleotide at that position. Since the DNA is typically amplified by emulsion PCR, the resulting bead, each containing only copies of the same DNA molecule, can be deposited on a glass slide resulting in sequences of quantities and lengths comparable to Illumina sequencing. A further method is based on Helicos' Heliscope technology, wherein fragments are captured by polyT oligomers tethered to an array. At each sequencing cycle, polymerase and single fluorescently labeled nucleotides are added and the array is imaged. The fluorescent tag is subsequently removed and the cycle is repeated. Further examples of sequencing techniques encompassed within the methods of the present invention are sequencing by hybridization, sequencing by use of nanopores, microscopy-based sequencing techniques, microfluidic Sanger sequencing, or microchip-based sequencing methods. The present invention also envisages further developments of these techniques, e.g. further improvements of the accuracy of the sequence determination, or the time needed for the determination of the genomic sequence of an organism etc.

According to one embodiment, the sequencing method comprises deep sequencing.

As used herein, the term "deep sequencing" refers to a sequencing method wherein the target sequence is read multiple times in the single test. A single deep sequencing run is composed of a multitude of sequencing reactions run on the same target sequence and each, generating independent sequence readout.

It will be appreciated that any of the analytical methods described herein can be embodied in many forms. For example, it can be embodied in on a tangible medium such as a computer for performing the method operations. It can be embodied on a computer readable medium, comprising computer readable instructions for carrying out the method operations. It can also be embodied in electronic device having digital computer capabilities arranged to run the computer program on the tangible medium or execute the instruction on a computer readable medium.

Computer programs implementing the analytical method of the present embodiments can commonly be distributed to users on a distribution medium such as, but not limited to, CD-ROMs or flash memory media. From the distribution medium, the computer programs can be copied to a hard disk or a similar intermediate storage medium. In some embodiments of the present invention, computer programs implementing the method of the present embodiments can be distributed to users by allowing the user to download the programs from a remote location, via a communication network, e.g., the internet. The computer programs can be run by loading the computer instructions either from their distribution medium or their intermediate storage medium into the execution memory of the computer, configuring the computer to act in accordance with the method of this invention. All these operations are well-known to those skilled in the art of computer systems.

Once the sequencing step is performed, a plurality of nucleic acid sequence data is obtained. The data includes the information from all the nucleic acid fragment reads which are obtained.

A "nucleic acid fragment read" as used herein refers to a single, short contiguous information piece or stretch of sequence data. A read may have any suitable length, preferably a length of between about 30 nucleotides to about 1000 nucleotides. The length generally depends on the sequencing technology used for obtaining it. In specific embodiments, the reads may also be longer, e.g. 2 to 10 kb or more. The present invention generally envisages any read or read length and is not to be understood as being limited to the presently available read lengths, but also includes further developments in this area, e.g. the development of long reading sequencing approaches etc.

The sequence data may additionally comprise information on the sequencing machine, date of acquisition, read length, direction of sequencing, origin of the sequenced entity, neighbouring sequences or reads, presence of repeats or any other suitable parameter known to the person skilled in the art. The sequence data may be presented in any suitable format, archive, coding or document known to the person skilled in the art. The data may, for example, be in the format of FASTQ, Qseq, CSFASTA, BED, WIG, EMBL, Phred, GFF, SAM, SRF, SFF or ABI-ABIF.

Preferably, the data or data sets are present in one data format, more preferably in a unified data format, e.g. in the fastq format, along with their base quality either in Phred/Phrap or modified format. It is further preferred that the data format at least covers the sequence read and its associated base quality.

In a particularly preferred embodiment of the present invention, the plurality of sequence data may be converted into a unified format. Such a conversion may be carried out by any suitable conversion tool known to the person skilled in the art, for example standard conversion tools which are capable of converting an Illumina format into a Sanger format, which may be used by several alignment algorithms, or any other comparable tool capable of converting a format into another format known to the person skilled in the art. The conversion may be performed such that at least a minimum amount of essential data is kept. Such a minimum amount of data may comprise, for example, the sequence itself, the run information, paired end library information, mate pair library information, single end library information, and base QC value. The preferred format into which the sequence data may be converted is any suitable format, which is recognized by reference sequence alignment algorithms, as well as de novo assembly algorithms. A preferred example is the fastq format. Alternatively, the sequence data may also be converted into the cfasta/SCARF format. The present invention further envisages any further, e.g. newly defined or developed format being able to be used by both, reference sequence alignments and de novo assembly procedures.

The data may comprise single entries or multiple entries within one data set. The data may also include one or more data sets, or a plurality of data sets. The term "plurality" as used herein accordingly refers to one or more data sets coming from one or more origins or sources. The data sets or data may, for example, have the same format and/or come from the same origin, e.g. the same sequencing machine, the same microbiome or have been obtained with the same sequencing technology, or they may have different formats and/or come from different origins such as different sequencing machines or different patients or subjects or have been obtained with different sequencing technologies.

Once the plurality of sequencing data has been obtained, the next step comprises aligning said plurality of nucleic acid sequence data to at least one reference sequence, said reference sequence being of the genome of the bacterium being analyzed.

The term "aligning to a reference sequence" as used herein refers to the comparison of nucleic acid fragment read information and their arrangement with an already existing genomic or sub-genomic sequence, preferably followed by a placement of said sequence read stretches within a scaffold provided by the preexisting genomic or sub-genomic sequence.

The "reference sequence" as used herein may be any suitable preexisting sequence covering the stretch, which is identical or similar to the newly obtained sequence data or nucleic acid fragment reads.

Bacterial genome sequences (reference sequences) may be derived from NCBI's microbial genome project database and other databases disclosed in Qin, J. et al. A metagenome-wide association study of gut microbiota in type 2 diabetes. Nature 490, 55-60 (2012), and Nielsen, H. B. et al. Identification and assembly of genomes and genetic elements in complex metagenomic samples without using reference genomes. Nat. Biotechnol. (2014). doi:10.1038/nbt.2939, incorporated herein by reference. Further details may be derived from McNeil L K et al., The National Microbial Pathogen Database Resource (NMPDR): a genomics platform based on subsystem annotation, Nucleic Acids Res., 2007; 35 (Database issue): D347-53.

The reference sequence may be essentially complete or comprise sub-portions of an essentially complete bacterial genome as defined below.

The term "essentially complete" as used herein refers to the presence of sequence information on all portions of the genome present in nature. For example, the genome sequence may comprise redundant sequences, repeats, telomeric sequences etc. For example, about 99%, 98%, 97%, 95%, 90%, 85%, 80%, or 75% of the genome sequence may be comprised in an essentially complete genome. In further embodiments, the reference sequence may not comprise certain sequence elements, such as repeats, telomeric sequences, transposon sequences, redundant sequences etc.

A "sub-portion" of an essentially complete genome may, for example, be any percentage of the entire genomic sequence, e.g. 10%, 20%, 30%, 40%, 60%, 65%, 70%, 75% etc. or any value in between. A sub-portion may also be a single chromosome sequence, a chromosomal arm, a combination of more than one chromosome, a haploid chromosomal set etc.

In a further preferred embodiment of the present invention the reference sequence as mentioned herein above may be selected from a group or taxon, which is phylogenetically related to the organism, whose nucleic acid data is to be assembled. Generally, a phylogenetically related organism may have an overall genomic identity of at least about 50%, 60%, 70%, 80%, 90% or 95%. A phylogenetically related organism may, for example, be a C. glutamticum in reference to E. coli sequence reads to be analyzed, or vice versa etc. In further embodiments, a reference sequence derived from a phylogenetically related organism may comprise a sub-portion of the entire genomic sequence, e.g. comprise certain chromosomes, chromosome combinations, chromosome arms, sections of the genome etc. as defined herein above.

The alignment to a reference sequence according to step (b) of the method according to the present invention may, in preferred embodiments, be carried out with or based on a suitable reference alignment algorithm. Preferred examples of such algorithms include the algorithms BFAST, ELAND, GenomeMapper, GMAP, MAQ, MOSAIK, PASS, SeqMap, SHRiMP, SOAP, SSAHA, or CLD. Particularly preferred is the use of the algorithms Bowtie or BWA. Further envisaged is the combination of one or more of these algorithms. For example, a reference alignment may first be carried out with one of the mentioned algorithms, followed by a repetition by a different of these algorithms. Results of both procedures may be compared and, where appropriate, combined. It is, in general, preferred to use contiguous nucleotide sequences showing a minimum number of non-matching reads or non-aligned reads.

Details and ways of implementing these algorithms would be known to the person skilled in the art, or can be derived from suitable literature sources, e.g. from Bao et al., Journal of Human Genetics, 28 Apr. 2011, p. 1-9. The present invention further envisages the use of optimized or further developed versions of these algorithms, or of reference alignment algorithms following a different scheme or algorithmic logic including not yet available algorithms, as long as the principle purpose of an alignment to a reference sequence as described herein is fulfilled.

Following alignment, the method continues by analyzing the frequency of at least one nucleotide positioned at the origin of replication of the genome and the frequency of at least one nucleotide positioned at the terminus of the genome, wherein the ratio of the frequencies is indicative of the growth dynamics of the bacterium.

As used herein the "origin of replication" refers to a particular sequence in a genome at which replication is initiated. The specific structure of the origin of replication varies somewhat from species to species, but all share some common characteristics such as high AT content (adenine and thymine). The origin of replication binds the pre-replication complex, a protein complex that recognizes, unwinds, and begins to copy DNA.

Most bacteria have a single circular molecule of DNA, and typically only a single origin of replication per circular chromosome.

The terminus of the genome is typically positioned approximately opposite the origin or replication on the circular bacterial genome.

The terminus region contains several DNA replication terminator sites, or "Ter" sites.

Analyzing the frequency of at least one nucleotide positioned at the origin of replication and the frequency of at least one nucleotide positioned at the terminus of the genome may be effected by analyzing the coverage pattern of the reads at these positions.

In another embodiment, the frequency of 20 % of the nucleotides across the genome of the bacterium is analyzed, wherein at least one of the nucleotides which is analyzed is positioned at the origin of replication and at least one of the nucleotides is positioned at the terminus.

In another embodiment, the frequency of 30 % of the nucleotides across the genome of the bacterium is analyzed, wherein at least one of the nucleotides which is analyzed is positioned at the origin of replication and at least one of the nucleotides is positioned at the terminus.

In another embodiment, the frequency of 40 % of the nucleotides across the genome of the bacterium is analyzed, wherein at least one of the nucleotides which is analyzed is positioned at the origin of replication and at least one of the nucleotides is positioned at the terminus.

In another embodiment, the frequency of 50 % of the nucleotides across the genome of the bacterium is analyzed, wherein at least one of the nucleotides which is analyzed is positioned at the origin of replication and at least one of the nucleotides is positioned at the terminus.

In another embodiment, the frequency of 60 % of the nucleotides across the genome of the bacterium is analyzed, wherein at least one of the nucleotides which is analyzed is positioned at the origin of replication and at least one of the nucleotides is positioned at the terminus.

In another embodiment, the frequency of 70 % of the nucleotides across the genome of the bacterium is analyzed, wherein at least one of the nucleotides which is analyzed is positioned at the origin of replication and at least one of the nucleotides is positioned at the terminus.

In another embodiment, the frequency of 80 % of the nucleotides across the genome of the bacterium is analyzed, wherein at least one of the nucleotides which is analyzed is positioned at the origin of replication and at least one of the nucleotides is positioned at the terminus.

In another embodiment, the frequency of 90 % of the nucleotides across the genome of the bacterium is analyzed, wherein at least one of the nucleotides which is analyzed is positioned at the origin of replication and at least one of the nucleotides is positioned at the terminus.

In another embodiment, the frequency of 95 % of the nucleotides across the genome of the bacterium is analyzed, wherein at least one of the nucleotides which is analyzed is positioned at the origin of replication and at least one of the nucleotides is positioned at the terminus.

It will be appreciated that if the position of the origin of replication and the terminus are known, then the method of this aspect of the present invention may be carried out by analyzing the coverage (or frequency) at these positions only. However, if the position of the origin of replication and the terminus are not known, it is preferable that essentially all (or the majority) of the nucleotides across the genome are analyzed. In this way, the position of the origin of replication and the terminus may be determined, as further described herein below.

Optionally, the frequencies of the nucleotides may be displayed graphically as a function of their genomic location. An exemplary graphic display is shown in Figures 2A and 2C herein. In these graphs, the peak coincides with the origin of replication and the trough coincides with the terminus.

The present inventors have shown that when the ratio of the frequency of a nucleotide at the origin of replication of the genome: frequency of the nucleotide at the terminus of the genome is about 2:1 or more, it is indicative of exponential growth of the bacterium.

Further, when the ratio of the frequency of a nucleotide at the origin of replication of the genome: frequency of a nucleotide at the terminus of the genome is about 1:1, it is indicative of stationary growth of the bacterium.

As mentioned, the method described herein above may be carried out in order to determine the origin of replication of a replication bacterium. This method comprises:

(a) sequencing DNA fragments of the genome of the bacterium to obtain a plurality of nucleic acid sequencing data;
(b) aligning said plurality of nucleic acid sequence data to a reference sequence, said reference sequence being of the genome of the bacterium; and
(c) analyzing the frequency of nucleotides across said genome of the bacterium; wherein the genomic location which corresponds to the highest number of reads is the origin of replication of the bacterium.

Steps (a) - (b) have been described herein above. In this case, analyzing the frequency of nucleotides is effected across the whole genome. It may be deduced that the genomic location which corresponds to the highest number of reads is the origin of replication of the bacterium.

As described above, the results of the frequency analysis may be presented graphically (see for example, Figures 2A and C). In this case, it may be deduced that the peak of the graph corresponds to the origin of replication, whereas the trough of the graph corresponds to the terminus.

According to one embodiment of this aspect of the present invention, the bacterium is comprised in a mixed population of bacteria and/or other microbes. The mixed population of bacteria may comprise more than 1, 2, 3, 4, 5, 10, 20 50, 100 or more species of microbes. According to a particular embodiment, the bacteria is comprised in a microbiome sample (e.g. gut microbiome) or any other microbiome disclosed herein.

It will be appreciated that as well as determining the position of the origin or replication of a bacteria, the present method enables the simultaneous analysis of growth dynamics, as further described herein above.

The present inventors have shown that the growth dynamics of bacteria of the microbiome can be used to assess the health thereof.

Thus, according to a further aspect of the present disclosure there is provided a method of analyzing the health of a test microbiome comprising:

(a) analyzing the growth dynamics of at least one bacterium in the test microbiome;
(b) comparing the growth dynamics of said at least one bacterium in the test microbiome to the growth dynamics of the at least one bacterium in a pathological microbiome, wherein when the growth dynamics of the at least one bacterium in the test microbiome is statistically significantly similar to the growth dynamics of the at least one bacterium in the pathological microbiome, it is indicative that the microbiome is not healthy.

As used herein, the term "pathological microbiome" refers to a microbiome derived from a subject who is known to have a disease (e.g. metabolic disease such as diabetes or pre-diabetes, ulcerative colitis, Crohn's disease, cancer or obesity).

It will be appreciated that microbiomes of the same source are compared (i.e. if the test microbiome is a gut microbiome, then the pathological microbiome is typically also a gut microbiome).

Microbiomes from all sources are contemplated such as those disclosed herein above.

Two bacteria can be classified as having statistically significantly similar growth dynamics if both are classified as stationary or both are classified as exponential.

According to one embodiment, analysis of growth dynamics is effected using the sequencing method described herein. Thus, for example if the frequency of the nucleotide at the origin of replication of the genome of the bacteria from the test microbiome : frequency of the nucleotide at the terminus of the genome of the bacteria from the test microbiome is about 2:1 or more and the frequency of the nucleotide at the origin of replication of the genome of the bacteria from the pathological microbiome : frequency of the nucleotide at the terminus of the bacteria from the pathological microbiome is about 2:1 or more, then the two bacteria may be considered as having statistically significant similar growth dynamics.

Further, if the frequency of the nucleotide at the origin of replication of the genome of the bacteria from the test microbiome : frequency of the nucleotide at the terminus of the genome of the bacteria from the test microbiome is about 1:1 and the frequency of the nucleotide at the origin of replication of the genome of the bacteria from the pathological microbiome : frequency of the nucleotide at the terminus of the bacteria from the pathological microbiome is about 1:1, then the two bacteria may be considered as having statistically significant similar growth dynamics.

Other methods of analyzing bacterial growth dynamics are known in the art and include for example analysis of optical density of a bacterial inoculant over a period of time.

In order to classify a test microbiome as being "pathological", typically at least 1, more preferably at least 5, more preferably at least 10, more preferably at least 20, more preferably at least 30, more preferably at least 40, more preferably at least 50, more preferably at least 100, more preferably at least 200, more preferably at least 300, more preferably at least 400, more preferably at least 500 of the bacteria therein have growth dynamics similar to those derived from a "pathological microbiome".

According to a particular embodiment, the growth dynamics of at least one of the following bacteria are analyzed to assess the health of a microbiome:

Shigella boydii - phylum P;
Shigella sonnei - phylum P;
Clostridium saccharolyticum - phylum F;
Bacteroides vulgates - phylum B;
Bacteroides thetaiotaomicron - phylum B;
Bifidobacterium longum - phylum A;
Streptococcus infantarius - phylum F;
Acidaminococcus intestine - phylum F;
Streptococcus anginosus - phylum F;
Parabacteroides distasonis - phylum B;
Odoribacter splanchnicus - phylum B;
Ruminococcus obeum - phylum F;
Streptococcus parasanguinis - phylum F;
Streptococcus sp. I-P16 - phylum F;
Streptococcus gordonii - phylum F;
Streptococcus sp. I-G2 - phylum F;
butyrate-producing bacterium SM4/1 - phylum F;
Shigella flexneri - phylum P;
Escherichia coli - phylum P;
Haemophilus parainfluenzae - phylum P;
butyrate-producing bacterium SS3/4 - phylum F;
Bifidobacterium adolescentis - phylum A;
Akkermansia muciniphila - phylum V;
Ruminococcus bromii - phylum F;
Coprococcus catus - phylum F;
butyrate-producing bacterium SSC/2 - phylum F;
Eubacterium rectal - phylum F;
Faecalibacterium prausnitzii - phylum F;
Roseburia intestinalis - phylum F;
Coprococcus sp. ART55/1 - phylum F;
Roseburia hominis - phylum F;
Ruminococcus torques - phylum F;
Bifidobacterium animalis - phylum A;
Eubacterium cylindroides - phylum F;
Alistipes shahii - phylum B;
Eubacterium siraeum - phylum F;
Eubacterium eligens - phylum F;
Lactobacillus sakei - phylum F;
Lactobacillus ruminis- phylum F;
Ruminococcus champanellensis - phylum F;
Adlercreutzia equolifaciens - phylum A; or
Bifidobacterium - phylum A.

According to a particular embodiment, at least 5 of the above mentioned bacteria are analyzed. According to a particular embodiment, at least 10 of the above mentioned bacteria are analyzed. According to a particular embodiment, at least 20 of the above mentioned bacteria are analyzed. According to a particular embodiment, at least 30 of the above mentioned bacteria are analyzed. According to a particular embodiment, all of the above mentioned bacteria are analyzed.

According to a particular embodiment, the growth dynamics of at least one, at least two, at least three, at least four, at least five, at least six or all of the following bacteria are analyzed to assess the health of a microbiome: Bifidobacterium longum, Shigella boydii, Shigella sonnei, Bacteroides vulgates, Bacteroides thetaiotaomicron, Acidaminococcus intestine and Parabacteroides distasonis.

According to still another embodiment, the mean growth dynamics of all of the bacteria in the test microbiome is compared with the mean growth dynamics of all of the bacteria in the pathological microbiome.

According to still another embodiment, the median growth dynamics of all of the bacteria in the test microbiome is compared with the median growth dynamics of all of the bacteria in the pathological microbiome.

It will be appreciated that as well as testing the growth dynamics of the bacteria in the microbiome, other bacterial parameters can also be analyzed such as abundance and presence of particular gene sequences.

Methods of quantifying levels of microbes of various types are described herein below.

In some embodiments, determining a level or set of levels of one or more types of microbes or components or products thereof comprises determining a level or set of levels of one or more DNA sequences. In some embodiments, one or more DNA sequences comprises any DNA sequence that can be used to differentiate between different microbial types. In certain embodiments, one or more DNA sequences comprises 16S rRNA gene sequences. In certain embodiments, one or more DNA sequences comprises 18S rRNA gene sequences. In some embodiments, 1, 2, 3, 4, 5, 10, 15, 20, 25, 50, 100, 1,000, 5,000 or more sequences are amplified.

In some embodiments, a microbiota sample (e.g. fecal sample) is directly assayed for a level or set of levels of one or more DNA sequences. In some embodiments, DNA is isolated from a microbiota sample and isolated DNA is assayed for a level or set of levels of one or more DNA sequences. Methods of isolating microbial DNA are well known in the art. Examples include but are not limited to phenol-chloroform extraction and a wide variety of commercially available kits, including QIAamp DNA Stool Mini Kit (Qiagen, Valencia, Calif.).

In some embodiments, a level or set of levels of one or more DNA sequences is determined by amplifying DNA sequences using PCR (e.g., standard PCR, semi-quantitative, or quantitative PCR). In some embodiments, a level or set of levels of one or more DNA sequences is determined by amplifying DNA sequences using quantitative PCR. These and other basic DNA amplification procedures are well known to practitioners in the art and are described in Ausubel et al. (Ausubel F M, Brent R, Kingston R E, Moore D D, Seidman J G, Smith J A, Struhl K (eds). 1998. Current Protocols in Molecular Biology. Wiley: New York).

In some embodiments, DNA sequences are amplified using primers specific for one or more sequence that differentiate(s) individual microbial types from other, different microbial types. In some embodiments, 16S rRNA gene sequences or fragments thereof are amplified using primers specific for 16S rRNA gene sequences. In some embodiments, 18S DNA sequences are amplified using primers specific for 18S DNA sequences.

In some embodiments, a level or set of levels of one or more 16S rRNA gene sequences is determined using phylochip technology. Use of phylochips is well known in the art and is described in Hazen et al. ("Deep-sea oil plume enriches indigenous oil-degrading bacteria." Science, 330, 204-208, 2010). Briefly, 16S rRNA genes sequences are amplified and labeled from DNA extracted from a microbiota sample. Amplified DNA is then hybridized to an array containing probes for microbial 16S rRNA genes. Level of binding to each probe is then quantified providing a sample level of microbial type corresponding to 16S rRNA gene sequence probed. In some embodiments, phylochip analysis is performed by a commercial vendor. Examples include but are not limited to Second Genome Inc. (San Francisco, Calif.).

In some embodiments, determining a level or set of levels of one or more types of microbes or components or products thereof comprises determining a level or set of levels of one or more microbial RNA molecules (e.g., transcripts). Methods of quantifying levels of RNA transcripts are well known in the art and include but are not limited to northern analysis, semi-quantitative reverse transcriptase PCR, quantitative reverse transcriptase PCR, and microarray analysis.

In some embodiments, determining a level or set of levels of one or more types of microbes or components or products thereof comprises determining a level or set of levels of one or more microbial polypeptides. Methods of quantifying polypeptide levels are well known in the art and include but are not limited to Western analysis and mass spectrometry. These and all other basic polypeptide detection procedures are described in Ausubel et al. In some embodiments, determining a level or set of levels of one or more types of microbes or components or products thereof comprises determining a level or set of levels of one or more microbial metabolites. In some embodiments, levels of metabolites are determined by mass spectrometry. In some embodiments, levels of metabolites are determined by nuclear magnetic resonance spectroscopy. In some embodiments, levels of metabolites are determined by enzyme-linked immunosorbent assay (ELISA). In some embodiments, levels of metabolites are determined by colorimetry. In some embodiments, levels of metabolites are determined by spectrophotometry.

It will be appreciated that as well as analyzing the health of a microbiome, the method described herein above may be used to diagnose a subject with having a disease.

Thus, according to still another aspect of the present invention there is provided a method of determining the health of a subject comprising:

(a) analyzing the growth dynamics of at least one bacteria in a microbiome sample of the subject;
(b) comparing said growth dynamics of said at least one bacteria in said microbiome sample of the subject to the growth rate of said at least one bacteria in at least one pathological microbiome, wherein when said growth rate of said at least one bacteria in said microbiome sample is statistically significantly similar to said growth rate of said at least one bacteria in said pathological microbiome, it is indicative that the subject is not healthy.

According to this aspect of the present invention, the subject from whom the test microbiome has been obtained can be diagnosed according to the state of his/her microbiome. If the test microbiome comprises bacteria which have growth rate which are similar to the growth rate of the corresponding bacteria in the pathological microbiome, it is indicative that the subject has a disease.

Alternatively, or additionally, if the test microbiome comprises bacteria which have growth dynamics which are similar to the growth dynamics of the corresponding bacteria in a healthy microbiome, it is indicative that the subject does not have a disease.

In order to diagnose a subject as having a disease, typically at least 1, more preferably at least 5, more preferably at least 10, more preferably at least 20, more preferably at least 30, more preferably at least 40, more preferably at least 50, more preferably at least 100, more preferably at least 200, more preferably at least 300, more preferably at least 400, more preferably at least 500 of the bacteria therein have growth rate similar to those derived from the pathological microbiome of that disease.

For example, when Eubacterium rectale of a test subject's gut microbiome has similar growth rate to Eubacterium rectale of a ulcerative colitis patient gut microbiome, this is indicative that the test subject has ulcerative colitis.

For example, when Butyrate-producing bacterium SS3/4 of a test subject's gut microbiome has similar growth rate to Butyrate-producing bacterium SS3/4 of a Crohn's patient gut microbiome, this is indicative that the test subject has Crohn's disease.

For example, when Eschericia Coli of a test subject's gut microbiome has similar growth rate to Eschericia Coli of a Crohn's patient gut microbiome, this is indicative that the test subject has Crohn's disease.

For example, when Bifidobacterium longum of a test subject's gut microbiome has similar growth rate to Bifidobacterium longum of a Crohn's patient or ulcerative colitis gut microbiome, this is indicative that the test subject has Crohn's disease or ulcerative colitis.

For example, analysis of the growth dynamics *Eggerthella lenta* of a test subjects gut microbiome can provide information as to whether a subject has an active form of Crohn's disease or is in remission.

When at least one of Klebsiella peneuoiae, Errerthella lenta, Escherichia coli, Butyrate producing bacterium SS3/4, Shigella boydii, Shigella sonnei, Bacteroides vulgates, Bacteroides thetaiotaomicron, Acidaminococcus intestine, Bifidobacerium adolscentis, Beiolonella parvula, Odoribacter splanchnicus, Bacteroides xylasisolvens, Alistipes shahii and Parabacteroides distasonis of a test subject's gut microbiome has similar growth rate to those bacteria of a Diabetes patient gut microbiome, this is indicative that the test subject has Diabetes.

According to a particular embodiment, in order to diagnose a subject the growth rate of at least one of the following bacteria are analyzed:

Shigella boydii - phylum P;
Shigella sonnei - phylum P;
Clostridium saccharolyticum - phylum F;
Bacteroides vulgates - phylum B;
Bacteroides thetaiotaomicron - phylum B;
Bifidobacterium longum - phylum A;
Streptococcus infantarius - phylum F;
Acidaminococcus intestine - phylum F;
Streptococcus anginosus - phylum F;
Parabacteroides distasonis - phylum B;
Odoribacter splanchnicus - phylum B;
Ruminococcus obeum - phylum F;
Streptococcus parasanguinis - phylum F;
Streptococcus sp. I-P16 - phylum F;
Streptococcus gordonii - phylum F;
Streptococcus sp. I-G2 - phylum F;
butyrate-producing bacterium SM4/1 - phylum F;
Shigella flexneri - phylum P;
Escherichia coli - phylum P;
Haemophilus parainfluenzae - phylum P;
butyrate-producing bacterium SS3/4 - phylum F;
Bifidobacterium adolescentis - phylum A;
Akkermansia muciniphila - phylum V;
Ruminococcus bromii - phylum F;

Coprococcus catus - phylum F;
butyrate-producing bacterium SSC/2 - phylum F;
Eubacterium rectal - phylum F;
Faecalibacterium prausnitzii - phylum F;
Roseburia intestinalis - phylum F;
Coprococcus sp. ART55/1 - phylum F;
Roseburia hominis - phylum F;
Ruminococcus torques - phylum F;
Bifidobacterium animalis - phylum A;
Eubacterium cylindroides - phylum F;
Alistipes shahii - phylum B;
Eubacterium siraeum - phylum F;
Eubacterium eligens - phylum F;
Lactobacillus sakei - phylum F;
Lactobacillus ruminis- phylum F;
Ruminococcus champanellensis - phylum F;
Adlercreutzia equolifaciens - phylum A; or
Bifidobacterium - phylum A.

According to another embodiment, in order to diagnose a subject, the growth rate of at least one of the following bacteria appearing in Figure 4 are analyzed.According to a particular embodiment, at least 5 of the above mentioned bacteria are analyzed. According to a particular embodiment, at least 10 of the above mentioned bacteria are analyzed. According to a particular embodiment, at least 20 of the above mentioned bacteria are analyzed. According to a particular embodiment, at least 30 of the above mentioned bacteria are analyzed. According to a particular embodiment, all of the above mentioned bacteria are analyzed.

According to a particular embodiment, the growth rate of at least one, at least two, at least three, at least four, at least five, at least six or all of the following bacteria are analyzed to assess the health of a subject: Bifidobacterium longum, Shigella boydii, Shigella sonnei, Bacteroides vulgates, Bacteroides thetaiotaomicron, Acidaminococcus intestine and Parabacteroides distasonis.

According to still another embodiment, the mean growth rate of all of the bacteria in the microbiome of the test subject is compared with the mean growth rate of all of the bacteria in the pathological microbiome.

According to still another embodiment, the median growth rate of all of the bacteria in the microbiome of the test subject is compared with the median growth rate of all of the bacteria in the pathological microbiome.

It is expected that during the life of a patent maturing from this application many relevant sequencing techniques will be developed and the scope of the term sequencing is intended to include all such new technologies *a priori.*

As used herein the term "about" refers to $\pm$ 10 %.

The terms "comprises", "comprising", "includes", "including", "having" and their conjugates mean "including but not limited to".

The term "consisting of' means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

It is appreciated that certain features of the invention, which are, for clarity, described in the context of separate embodiments, may also be provided in combination in a single embodiment that falls under the scope of the appended claims. Conversely, various features of the invention, which are, for brevity, described in the context of a single embodiment, may also be provided separately or in any suitable subcombination or as suitable in any other described embodiment of the invention as defined in the appended claims. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

## EXAMPLES

Reference is now made to the following examples, which together with the above descriptions illustrate some embodiments of the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996). Other general references are provided throughout this document. The procedures therein are believed to be well known in the art and are provided for the convenience of the reader.

## MATERIALS AND METHODS

*Ex-vivo E. coli culture and sequencing.* Batch cultures of E.coli cells (K-12 strain) were grown in LB medium from a frozen stock. The cultures were cultivated at 37oC with shaking at 200rpm and cells were harvested after 24 hours at O.D.600 of 9 for the purification of DNA from a stationary culture. In order to produce DNA from an exponentially growing culture, an over-night starter culture was diluted 1:600, grown for 3 hours and the cells were harvested at O.D.600 of 0.2. DNA was purified using DNeasy Blood & Tissue Kit (Qiagen). For shotgun sequencing, 1$\mu$g of purified genomic DNA was sheared with a Covaris M220 sonicator. Illumina compatible libraries were then prepared as described (Suez et al.) and sequenced on a HiSeq 2500 machine.

*Data.* Replication origin locations were obtained from Gao, F., Luo, H. & Zhang, C.-T. DoriC 5.0: an updated database of oriC regions in both bacterial and archaeal genomes. Nucleic Acids Res. 41, D90-3 (2013). Complete bacterial genomes were obtained as follows. First, complete genomes were downloaded (March 2014) from RefSeq[36] and from Ensembl[37]. Genomes that were incomplete (fragments, super-contigs, etc.) or plasmids were filtered, and duplicate genomes were removed. Metagenomic datasets were obtained from ref.[7] (363 samples) and ref.[9] (346 samples; only samples with complete metadata were retrieved).

*Sequencing coverage analysis.* Samples were mapped to a database containing full bacterial genomes using GEM mapper[38] at paired-end mode with specific parameters (-q offset-33 -gem-quality-threshold 26). Reads mapped to multiple species were correctly assigned and/or split if needed using an expectation-maximization (EM) algorithm adapted from Pathoscope[39]. In the common case of reads mapped to different strains of the same species, a representative strain was chosen for each sample as the strain with the highest abundance. The total number of sequencing reads that mapped to each bacteria was summed into non-overlapping 10Kbp bins for display purposes. We then employed a smoothing filter, comprised of a moving sum with window size of 10Kbp and a slide of 100bp, followed by a moving median with window size of 10K bins and a slide of a 100 bins. Bins that were not within an 8-fold range symmetrically surrounding the median bin coverage across the entire bacterial genome were discarded, and bacteria with more than 40% of its bins discarded or with less than 10 remaining bins were discarded as well.

*Predictions of replication origin locations.* The Levenberg-Marquardt algorithm for non-linear least squares minimization, implemented by lmfit[40], was used to fit the prototypical coverage function to the smoothed coverage bins of each bacteria in each sample. The coverage function is a segmentally linear function between the peak and trough location and coverage ($ORI_{loc}$, $ORI_{cov}$) and ($TER_{loc}$, $TER_{cov}$):

$$f(x) = \begin{cases} -ax + y_1 + ax_1 & x \le x_1 \\ ax + y_1 - ax_1 & x_1 < x < x_2 \\ -ax + y_2 - ax_2 & x_2 \le x \end{cases}$$

Where:

$$a = \frac{TER_{cov} - ORI_{cov}}{TER_{loc} - ORI_{loc}}$$

$$x_1 = \min(TER_{loc}, ORI_{loc}) \quad y_1 = \begin{cases} TER_{cov} & if \ x_1 = TER_{loc} \\ ORI_{cov} & if \ x_1 = ORI_{loc} \end{cases}$$

$$x_2 = \max(TER_{loc}, ORI_{loc}) \quad y_2 = \begin{cases} TER_{cov} & if \ x_2 = TER_{loc} \\ ORI_{cov} & if \ x_2 = ORI_{loc} \end{cases}$$

$ORI_{loc}$ and $TER_{loc}$ were constrained to be separated by 45-55% of the bacterial genome length. p-values were assigned by permutations of the coverage bins, and only fits with $p < 0.05$ and peak-to-trough ratio ($ORI_{cov}/TER_{cov}$) larger than 1.1 were retained.

Predictions of replication origin location were done only for bacteria that had successful coverage fits in at least 3 different samples. The replication origin location was predicted as the circular-median of the $ORI_{loc}$ of the bacteria across different samples. Circular median was designed to handle the case in which the replication origin is at the edge of the given genome sequence, in which case coverage peaks of the same bacteria across different samples might be located at both edges of the genome, and thus a regular median would falsely determine the location to be in the middle of the sequence. It is defined as:

$$circular\ median(\overline{p}, g) = \big(median\big((\overline{p} - t_m) \bmod g\big) + t_m\big) \bmod g$$

where

$$t_m = \operatorname*{argmin}_{t \in \overline{p}} \big\{ \max\big((\overline{p} - t) \bmod g\big) - \min\big((\overline{p} - t) \bmod g\big) \big\}$$

Where $g$ is the genome length and $\vec{p}$ the vector of different sample coverage peaks. Prediction of replication terminus location was done in a similar manner.

*Peak-to-trough ratio.* Peak-to-trough ratios were calculated per bacteria in each sample as the smoothed sequencing coverage (see above section on sequencing coverage analysis) at the predicted peak location divided by that at the predicted trough location. Two conditions need to be met for a ratio to be calculated: (1) Replication origin and terminus locations were predicted for the given bacteria (i.e., there were at least 3 samples in which the coverage function was fitted with $p<0.05$ and with fitted peak-to-trough ratio > 1.1); (2) The bacteria had sufficient coverage in the given sample, and was retained after the smoothing filter was employed as part of the sequencing coverage analysis.

*Correlation of peak-to-trough ratios and clinical parameters.* Correlations were computed for each dataset[7,9] separately. Spearman correlations were calculated independently between peak-to-trough ratios, relative abundances, and peak-to-trough ratios after linear correction for relative abundance; and different host variables. Only bacteria that had peak-to-trough ratios calculated in over 20 samples were taken into consideration in this analysis. Missing microbial data was masked and not included in the correlation. Host variables included were BMI, prevalence of Type II Diabetes, fasting blood glucose, free insulin, HbA1C% and weight for Qin et. al[7]; and BMI and prevalence of Crohn's Disease (CD) and Ulcerative Colitis (UC) for MetaHIT[9]. For the latter, correlation with UC or CD prevalence was done only for the samples belonging to Spanish individuals, as only this subset contained inflicted individuals. In addition, correlation with BMI was calculated for each nationality (Danish, Spanish) separately. All correlations were FDR corrected for multiple hypothesis testing.

*Correlation between literature and predicted origin locations.* Correlation in Figure 2E is Pearson correlation. When comparing the predicted origin replication location to that found in the literature for a given bacteria, its genome length was added to the smallest location in cases where the error was larger than 50% of the genome length, to account for the circularity of the bacterial genome.

## EXAMPLE 1

### *Coverage analysis uncovers a prototypical pattern in E. Coli in both ex-vivo cultures and in vivo metagenomic samples*

As proof of concept of the idea that analysis of sequencing coverage may be informative of DNA replication and growth dynamics, ex-vivo cultures of *E. Coli* (K-12 strain) were grown. They were sampled in either the exponential growth phase or the stationary growth phase (see Methods). DNA was extracted from each sample, wherein each resulting DNA pool was submitted to next-generation sequencing, and the coverage patterns of the reads were analyzed after mapping them to the *E. coli* genome. Indeed, in stationary phase, where most of the cells in the culture were not growing and thus had a single copy of their genome, uniform coverage across the genome was found (Figures 1A, B). In contrast, the coverage pattern of the sample taken from the exponential growth phase, in which many bacteria cells were likely engaged at different stages of DNA replication, exhibited a single trough and a single peak, with the peak coinciding with the known[30] replication origin *of E. coli* (Figure 1B). This suggests that the coverage pattern stems from the different copy number that each bacteria in the population has for the different parts of its genome, reflective of its DNA replication stage. It was further noted that the ratio between the coverage at the peak and that at the trough was ~3:1 in the exponential growth sample compared to ~1:1 in the stationary phase sample, suggesting that these ratios are representative of the growth dynamics of the two cultures.

Notably, the same prototypical patterns were found when performing the same coverage analysis of the *E. coli* genome on 522 publicly available[7,9] in-vivo metagenomic samples from human stool in which the bacteria was present in sufficient abundance, with the coverage peak and trough locations coinciding with their locations in the ex-vivo cultures (Figure 1C). Moreover, the peak-to-trough ratios varied greatly across a range of 0.97-3.55, which is in striking agreement with the range of *E. coli* growth dynamics that span stationary phase growth (ratio=1.1) and exponential phase growth in culture (ratio=3.3, Figure 1C).

Taken together, these results suggest that the process of DNA replication generates a prototypical signature that can be detected through coverage analysis of sequencing reads. Moreover, the ratio between the peak and trough coverage across the bacterial genome varies greatly across different conditions in culture and across different human microbiome samples, with higher ratios likely indicative of higher replication rate and thus faster growth of the analyzed bacterial population.

## EXAMPLE 2

### *Coverage analysis accurately identifies replication origins*

To generalize the present approach to a mixed microbial population and test whether coverage pattern analysis applies to species other than *E. coli,* the present inventors applied the analysis to all of the bacteria for which complete genomes (Methods) are available and across 346 publicly available metagenomic stool samples[9]. Notably, they found prototypical coverage patterns similar to those of *E. coli* across 168 different bacteria, with the pattern of each bacteria consisting of a single peak and a single trough (Figures 2A, C). Moreover, for each bacteria, the coverage patterns across different samples exhibited good agreement in the locations of the peaks and troughs across different human microbiome samples (Figures 2B, D).

To test whether as in *E. coli,* the location of the peak coverage in every bacteria corresponds to the location of its origin of replication, the present inventors computed, for each of the above 168 bacteria, the median (circular-median, see Methods) of its peak locations across different samples. Indeed, these median locations, computed solely based on the present analysis of the bacterial coverage patterns, were in excellent agreement with the replication origins of 113 different bacteria whose origin is already known[30] (Pearson-$R^2$=0.98, $p<10^{-98}$, Figure 2E). For example, for *Parabacteroides distasonis,* the predicted origin location was ~180kb away from the known origin, corresponding to a difference of only ~3.7% of the bacteria's genome length. Following closer inspection of the few cases that had a larger disagreement between the median peak locations and the known origins, the known origin location is probably misplaced in 6 bacteria and corrected locations are suggested herein (Figures 5A-F). For example, in *Odoribacter splanchius*, the known origin is located at the start of the genome, whereas our analysis based on 72 human microbiome samples identified a prototypical peak and trough pattern at position 3.3Mbp. Notably, for 55 of the 168 bacteria for which we performed our coverage analysis, the origin location is unknown and our analysis thus offers novel origin locations (Figures 2F, 6A-I).

This good correspondence that we found across most bacteria between their known replication origins and their peak locations, computed solely by applying coverage analysis to metagenomic samples, provides further evidence that the prototypical coverage patterns that were uncovered are indeed informative of the DNA replication process that the corresponding bacteria are undergoing in their embedding host. Moreover, it shows that this coverage analysis may uncover structural properties of bacterial genomes, by identifying novel putative replication origins for bacteria with unknown origins, and possibly correcting misplaced origin locations.

## EXAMPLE 3

### *Peak-to-trough ratios exhibit diurnal oscillations out of phase with abundance oscillations*

The present inventors next aimed to determine the physiological relevance of coverage analysis for assessing the behavioral patterns of distinct species within a microbiome configuration. In recent work, it was found that -15% of the microbial members of the intestinal microbiota exhibit diurnal oscillations, manifested in rhythmic changes in abundance and functions of these 'driver' bacteria over the course of a day. It was further demonstrated that these microbial rhythms bear fundamental importance to host-microbiome interactions, and that diurnal disturbances in microbiome rhythms drive host susceptibility to obesity and glucose intolerance.

As these diurnal oscillatory changes in bacteria abundances likely result from within-day changes in their proliferation rate, the present inventors employed their coverage analysis method to these data, hypothesizing that the rhythmic changes in bacterial proliferation would manifest themselves in changes in their peak-to-trough coverage ratios across different times of the day. To this end, they analyzed metagenomic stool samples that were obtained from two different human individuals approximately every 6 hours for a total of 4 or 5 consecutive days. Application of the commonly used JTK cycle[32] algorithm identified 6 bacteria whose abundance levels cycled with a periodicity of 24 hours and that had sufficient abundance levels across the different samples to allow for our peak-to-trough coverage analysis. Notably, in all 6 bacteria the peak-to-trough ratio across samples also exhibited oscillatory patterns and in 3 cases these oscillations were statistically significant with a 24-hour periodicity ($P<0.05$, Figures 3A-F). Moreover, the oscillations of 5 out of 6 bacteria were out of phase with the oscillations in relative abundance levels (Figures 3A-F). For example, for *Ruminococcus obeum*, the peak-to-trough ratios are highest every day at Zeitgeber time (ZT) of 12 hours, whereas the abundance of that bacteria is highest only 6 hours later, at ZT of 18 hours (Figure 3A). This anti-correlation between bacteria abundance and peak-to-trough ratios is not a trivial property of these two measures whereby one measure is high when the other is low, because across different human samples, these two measures are not correlated (Figure 7).

Notably, for 2 of the above 6 bacteria (*R. obeum* and *Eubacterium siraeum*) there are no known origins of replication in the literature[30], yet the present analysis predicted novel origins that produced cycling peak-to-trough ratios when used in the coverage analysis (Figures 3A, B). This provides further independent support for the correctness of our putative origins and the utility of our approach. In other cases, such as for *P. distasonis* and *Haemophilus parainfluenzae* the origins identified by the coverage analysis agreed with the literature origins and their peak-to-trough ratios also exhibited diurnal oscillations (Figures 3C, D).

These results provide further support for the idea that peak-to-trough ratios reflect bacterial growth dynamics, and suggest that diurnal changes in the abundance of some bacteria result from diurnal changes in their proliferation rates. More globally, the present results suggest that an increase in the abundance of a bacteria within the microbiota ecosystem may be preceded in time and thus predictable by an increase in the bacteria's peak-to-trough ratio.

## EXAMPLE 4

### *Bacterial peak-to-trough ratios correlate with multiple host clinical parameters*

To test whether bacterial PTRs are associated with disease and different clinical parameters, PTRs were generated for every species in samples from European (N=396) and Chinese (N=363) cohorts. In both datasets, a large variation in PTRs was found across samples (Figure 4). Notably, statistically significant associations were found between PRTs of 20 different bacteria ad multiple clinical parameters, including significant correlations between the PTR of Bifidobacterium longum and occurrence of Crohn's disease in the Spanish national of the European cohort (FDR-corrected Mann-Whitney P is less than 0.005) Figure 4, and between the PTRs of 12 different bacteria and the occurrence of type II diabetes in the Chinese cohort. Significant correlations between PTRs and the occurrence of ulcerative colitis, body mass index (BMI), the fraction of glycated hemoglobin, fasting serum insulin and fasting blood glucose levels.

These associations are independent of- and unobtainable by examining bacterial abundances, as: (1) in correlating PTRs with clinical parameters only samples in which that bacteria was present was used, thereby withholding information about the presence or absence of the examined bacteria; (2) in only 5 of the 38 statistically significant correlations were the abundance levels also correlated with the same clinical parameter; and 36 or the 38 significant associations of PTR remained significant after correlating them for relative abundance levels. The PTR of some species were correlated with clinical parameters only after correlation for relative abundance including Eubacterium rectal and the occurrence of Crohn's disease (FDR corrected Mann-Whitney P is less than $10^{-4}$).

As a global measure of the growth dynamics of the entire microbiota, for every sample, both the mean and median of the PTRs of all of the bacteria present was calculated. This global measure correlated with fasting glucose and HbA1c% levels and with the occurrence of Crohn's disease and type II diabetes, indicating that global microbiome growth dynamics also associate with disease (Figure 4).

A preliminary analysis of 40 samples from the Prospective Registry in IBD study of MGH (PRISM) cohort showed that only 4 bacteria passed the present stringent pipeline filters for PTR calculation in more than half of the samples. Notwithstanding, *Eggerthella lenta* presented significantly different PTRs between patients with active Crohn's disease and patients in remission (FDR corrected Mann-Whitney P is less than 0.1). Neither the abundance of *E. lenta*, nor of the other three species differed.

Between active and quiescent Crohn's patients, highlighting the fact that PTRs reflect an independent feature of the effect of the microbiome on its host.

Taken together, these results show that peak-to-trough coverage ratios contain clinically relevant information, and that this information is independent of that contained in the bacteria abundance levels, suggesting that for some bacteria, its growth dynamics within the ecosystem may be more relevant than its relative abundance level.

## Conclusions and discussion

In summary, the examples above show that the pattern of read coverage of bacteria within metagenomic samples can be used as a new modality for elucidating growth kinetic behaviors of microbiome bacterial members, inferred from a single static metagenomic sample. In applying coverage analysis to 709 human gut metagenomic samples[7,9], the present inventors demonstrate that most bacteria exhibit a prototypical coverage pattern, consisting of a single peak and a single trough across their genome. Multiple lines of evidence are presented herein demonstrating that these coverage patterns likely reflect growth dynamics of discrete bacterial members of the microbiota ecosystem. First, for most bacteria whose replication origin is known, the coverage pattern peaks very close to the origin, matching what would be expected from a signature of bacterial growth and replication. Second, the range of values of the peak-to-trough ratios across different human microbiome samples falls between the ratios observed for a non-growing bacterial population (in stationary phase) and an exponential-growth bacterial population. Third, in a setting in which bacteria change their abundance within hours, corresponding changes in their peak-to-trough coverage ratios that are out of phase with the changes in abundance are observed, reflecting an increase in the peak-to-trough ratio that temporally precedes the increase in bacteria abundance.

The present results demonstrate that this novel feature, extractable by simple analysis of metagenomic data, can provide new insights into microbiota structure and function. First, for bacteria whose origin of replication is unknown, coverage analysis can suggest putative origin locations (as we show here for 55 bacteria) and for other bacteria it may help in suggesting corrections to misplaced origins (shown here for 6 bacteria). Second, the finding that changes in the peak-to-trough ratio may precede changes in abundance suggests that coverage analysis may be useful for predicting future changes in the microbiota composition. Finally, many associations between peak-to-trough ratios of bacteria and several important clinical parameters were found, such as prevalence of disease (e.g., Crohn and type II diabetes mellitus). Notably, the abundance of these same bacteria are not associated with the same clinical parameters, strongly suggesting that for some bacteria, their growth dynamics is a more clinically relevant measure that their relative abundance within the ecosystem.

Notably, these findings were obtained from metagenomic samples of stool that represent a very complex ecological niche, which is further complicated by the fact that the bacterial communities are unsynchronized and that the genomes of many of the bacteria differ to varying degrees from the currently available reference bacterial genomes.

Utilizing read coverage analysis to 'fish out' discrete microbial kinetic behavior in a complex microbiome population has multiple potential clinical implications. It may extend our understanding of host-microbiota interactions from the current static view to a dynamic one, in which the microbiota may be regarded as a flexible functional unit, highly responsive to environmental signals. As such, uncovering individual bacterial proliferation patterns within a highly complex ecosystem may enable to identify active 'driver' and 'modulator' species, which are most responsive to changing environmental conditions from among the thousands of bystander commensal species. It may enable to pinpoint disease-causing or disease-modulating microbes contributing to multi-factorial diseases, whose inherent proliferation activity may be masked by variations in abundance. Furthermore, the present method may be able to detect, follow, and assess therapeutic responsiveness of pathogens or pathobionts introduced into the ecosystem, which are expected to be highly proliferative during their virulent state, yet may be hard to differentiate from among similar non-virulent commensal bacterial species.

Overall, a new type of metagenomic data analysis is presented that provides a view of the growth dynamics of the microbiota from a single snapshot sample, which may be of clinical relevance. As such, coverage analysis introduces a new feature to a much needed 'functional toolkit' enabling to exploit the vast data generated by shotgun metagenomic analysis to infer functional microbial properties that can aid in the quest for a mechanistic understanding of the role of host-microbiota interactions in shaping homeostasis and disease susceptibility.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

In addition, citation or identification of any reference in this application shall not be construed as an admission that such reference is available as prior art to the present invention. To the extent that section headings are used, they should not be construed as necessarily limiting.

REFERENCES

1. Qin, J. et al. A human gut microbial gene catalogue established by metagenomic sequencing. Nature 464, 59-65 (2010).

2. Rho, M., Tang, H. & Ye, Y. FragGeneScan: predicting genes in short and error-prone reads. Nucleic Acids Res. 38, e191 (2010).

3. Human, T. & Project, M. Structure, function and diversity of the healthy human microbiome. Nature 486, 207-14 (2012).

4. Turnbaugh, P. J. et al. A core gut microbiome in obese and lean twins. Nature 457, 480-4 (2009).

5. Markowitz, V. M. et al. IMG/M-HMP: a metagenome comparative analysis system for the Human Microbiome Project. PLoS One 7, e40151 (2012).

6. Meyer, F. et al. The metagenomics RAST server - a public resource for the automatic phylogenetic and functional analysis of metagenomes. BMC Bioinformatics 9, 386 (2008).

7. Qin, J. et al. A metagenome-wide association study of gut microbiota in type 2 diabetes. Nature 490, 55-60 (2012).

8. Karlsson, F. H. et al. Gut metagenome in European women with normal, impaired and diabetic glucose control. Nature 498, 99-103 (2013).

9. Nielsen, H. B. et al. Identification and assembly of genomes and genetic elements in complex metagenomic samples without using reference genomes. Nat. Biotechnol. (2014). doi:10.1038/nbt.2939

10. Schloissnig, S. et al. Genomic variation landscape of the human gut microbiome. Nature 493, 45-50 (2013).

11. Urban, A. E. et al. High-resolution mapping of DNA copy alterations in human chromosome 22 using high-density tiling oligonucleotide arrays. Proc. Natl. Acad. Sci. U. S. A. 103, 4534-9 (2006).

12. Bailey, J. A. et al. Recent segmental duplications in the human genome. Science 297, 1003-7 (2002).

13. Cheng, Z. et al. A genome-wide comparison of recent chimpanzee and human segmental duplications. Nature 437, 88-93 (2005).

14. Chiang, D. Y. et al. High-resolution mapping of copy-number alterations with massively parallel sequencing. Nat. Methods 6, 99-103 (2009).

15. Alkan, C. et al. Personalized copy number and segmental duplication maps using next-generation sequencing. Nat. Genet. 41, 1061-7 (2009).

16. Campbell, P. J. et al. Identification of somatically acquired rearrangements in cancer using genome-wide massively parallel paired-end sequencing. Nat. Genet. 40, 722-9 (2008).

17. McKernan, K. J. et al. Sequence and structural variation in a human genome uncovered by short-read, massively parallel ligation sequencing using two-base encoding. Genome Res. 19, 1527-41 (2009).

18. Yoon, S., Xuan, Z., Makarov, V., Ye, K. & Sebat, J. Sensitive and accurate detection of copy number variants using read depth of coverage. Genome Res. 19, 1586-92 (2009).

19. Medvedev, P., Fiume, M., Dzamba, M., Smith, T. & Brudno, M. Detecting copy number variation with mated short reads. Genome Res. 20, 1613-22 (2010).

20. Abyzov, A., Urban, A. E., Snyder, M. & Gerstein, M. CNVnator: an approach to discover, genotype, and characterize typical and atypical CNVs from family and population genome sequencing. Genome Res. 21, 974-84 (2011).

21. Xu, J. et al. Genome-wide identification and characterization of replication origins by deep sequencing. Genome Biol. 13, R27 (2012).

22. Skovgaard, O., Bak, M., Løbner-Olesen, A. & Tommerup, N. Genome-wide detection of chromosomal rearrangements, indels, and mutations in circular chromosomes by short read sequencing. Genome Res. 21, 1388-93 (2011).

23. Allardet-Servent, A., Michaux-Charachon, S., Jumas-Bilak, E., Karayan, L. & Ramuz, M. Presence of one linear and one circular chromosome in the Agrobacterium tumefaciens C58 genome. J. Bacteriol. 175, 7869-74 (1993).

24. Hinnebusch, J. & Tilly, K. Linear plasmids and chromosomes in bacteria. Mol. Microbiol. 10, 917-22 (1993).

25. Wang, J. D. & Levin, P. A. Metabolism, cell growth and the bacterial cell cycle. Nat. Rev. Microbiol. 7, 822-7 (2009).

26. Cooper, S. & Helmstetter, C. E. Chromosome replication and the division cycle of Escherichia coli B/r. J. Mol. Biol. 31, 519-40 (1968).

27. SCHAECHTER, M., MAALOE, O. & KJELDGAARD, N. O. Dependency on medium and temperature of cell size and chemical composition during balanced grown of Salmonella typhimurium. J. Gen. Microbiol. 19, 592-606 (1958).

28. Fossum, S., Crooke, E. & Skarstad, K. Organization of sister origins and replisomes during multifork DNA replication in Escherichia coli. EMBO J. 26, 4514-22 (2007).

29. Nielsen, H. J., Youngren, B., Hansen, F. G. & Austin, S. Dynamics of Escherichia coli chromosome segregation during multifork replication. J. Bacteriol. 189, 8660-6 (2007).

30. Gao, F., Luo, H. & Zhang, C.-T. DoriC 5.0: an updated database of oriC regions in both bacterial and archaeal genomes. Nucleic Acids Res. 41, D90-3 (2013).

31. Morgan, X. C. & Huttenhower, C. Chapter 12: Human microbiome analysis. PLoS Comput. Biol. 8, e1002808 (2012).

32. Hughes, M. E., Hogenesch, J. B. & Komacker, K. JTK_CYCLE: an efficient nonparametric algorithm for detecting rhythmic components in genome-scale data sets. J. Biol. Rhythms 25, 372-80 (2010).

33. Le Chatelier, E. et al. Richness of human gut microbiome correlates with metabolic markers. Nature 500, 541-6 (2013).

34. Ahn, J. et al. Human gut microbiome and risk for colorectal cancer. J. Natl. Cancer Inst. 105, 1907-11 (2013).

35. Yoshimoto, S. et al. Obesity-induced gut microbial metabolite promotes liver cancer through senescence secretome. Nature 499, 97-101 (2013).

36. Tatusova, T., Ciufo, S., Fedorov, B., O'Neill, K. & Tolstoy, I. RefSeq microbial genomes database: new representation and annotation strategy. Nucleic Acids Res. 42, D553-9 (2014).

37. Flicek, P. et al. Ensembl 2014. Nucleic Acids Res. 42, D749-55 (2014).

38. Marco-Sola, S., Sammeth, M., Guigó, R. & Ribeca, P. The GEM mapper: fast, accurate and versatile alignment by filtration. Nat. Methods 9, 1185-8 (2012).

39. Francis, O. E. et al. Pathoscope: species identification and strain attribution with unassembled sequencing data. Genome Res. 23, 1721-9 (2013).

40. Newville, M., Ingargiola, A., Stensitzki, T. & Allen, D. B. LMFIT: Non-Linear Least-Square Minimization and Curve-Fitting for Python. (2014). doi:10.5281/zenodo.11813

## Claims

1. A method of diagnosing a disease of a subject comprising:

   (a) sequencing DNA fragments of at least one bacterium in a microbiome sample of the subject to obtain a plurality of nucleic acid sequencing data;
   (b) aligning said plurality of nucleic acid sequence data to at least one reference sequence, said reference sequence being of a genome of the bacterium; and
   (c) analyzing the frequency of at least one nucleotide positioned at the origin of replication of said genome and the frequency of at least one nucleotide positioned at the replication terminus of said genome, wherein the ratio of said frequencies is indicative of the growth rate of the bacterium;
   (d) comparing said growth rate of said at least one bacterium in said microbiome sample of the subject to the growth rate of said at least one bacterium in a microbiome of a subject having said disease, wherein when said growth rate of said at least one bacterium in said microbiome sample is statistically significantly similar to said growth rate of said at least one bacterium in said microbiome of said subject having said disease, it is indicative that the subject has said disease.

2. The method of claim 1, wherein said at least one bacterium is selected from the group consisting of those set forth in Figure 4.

3. The method of claim 1, wherein said disease is a metabolic disorder.

4. The method of claim 3, wherein said metabolic disorder is selected from the group consisting of Diabetes, obesity, ulcerative colitis and Crohn's disease.

**Patentansprüche**

1. Verfahren zur Diagnose einer Krankheit eines Subjekts, Folgendes umfassend:

(a) Sequenzieren von DNA-Fragmenten von zumindest einem Bakterium in einer Mikrobiomprobe des Subjekts, um eine Vielzahl von Nukleinsäuresequenzierungsdaten zu erhalten;
(b) Ausrichten der Vielzahl von Nukleinsäuresequenzdaten auf zumindest eine Referenzsequenz, wobei die Referenzsequenz von einem Genom des Bakteriums stammt; und
(c) Analysieren der Häufigkeit von zumindest einem Nukleotid, das am Replikationsursprung des Genoms positioniert ist, und der Häufigkeit von zumindest einem Nukleotid, das am Replikationsende des Genoms positioniert ist, wobei das Verhältnis der Häufigkeiten die Wachstumsrate des Bakteriums anzeigt;
(d) Vergleichen der Wachstumsrate des zumindest einen Bakteriums in der Mikrobiomprobe des Subjekts mit der Wachstumsrate des zumindest einen Bakteriums in einem Mikrobiom eines Subjekts, das die Krankheit aufweist, wobei, wenn die Wachstumsrate des zumindest einen Bakteriums in der Mikrobiomprobe statistisch signifikant ähnlich der Wachstumsrate des zumindest einen Bakteriums in dem Mikrobiom des Subjekts, das die Krankheit aufweist, ist, es anzeigt, dass das Subjekt die Krankheit aufweist.

2. Verfahren nach Anspruch 1, wobei das zumindest eine Bakterium ausgewählt ist aus der Gruppe bestehend aus den in Figur 4 dargestellten.

3. Verfahren nach Anspruch 1, wobei die Krankheit eine Stoffwechselstörung ist.

4. Verfahren nach Anspruch 3, wobei die Stoffwechselstörung ausgewählt ist aus der Gruppe bestehend aus Diabetes, Fettleibigkeit, Colitis ulcerosa und Morbus Crohn.

**Revendications**

1. Procédé de diagnostic d'une maladie d'un sujet comprenant :

(a) le séquençage de fragments d'ADN d'au moins une bactérie dans un échantillon de microbiome du sujet pour obtenir une pluralité de données de séquençage d'acides nucléiques ;
(b) l'alignement de ladite pluralité de données de séquence d'acides nucléiques sur au moins une séquence de référence, ladite séquence de référence étant d'un génome de la bactérie ; et
(c) l'analyse de la fréquence d'au moins un nucléotide positionné à l'origine de réplication dudit génome et la fréquence d'au moins un nucléotide positionné au terminus de réplication dudit génome, le rapport desdites fréquences indiquant le taux de croissance de la bactérie ;
(d) la comparaison dudit taux de croissance de ladite au moins une bactérie dans ledit échantillon de microbiome du sujet au taux de croissance de ladite au moins une bactérie dans un microbiome d'un sujet atteint de ladite maladie, dans lequel, lorsque ledit taux de croissance de ladite au moins une bactérie dans ledit échantillon de microbiome est statistiquement significativement similaire audit taux de croissance de ladite au moins une bactérie dans ledit microbiome dudit sujet ayant ladite maladie, cela indique que le sujet est atteint de ladite maladie.

2. Procédé selon la revendication 1, dans lequel ladite au moins une bactérie est sélectionnée dans le groupe constitué de celles présentées à la figure 4.

3. Procédé selon la revendication 1, dans lequel ladite maladie est un trouble métabolique.

4. Procédé selon la revendication 3, dans lequel ledit trouble métabolique est sélectionné dans le groupe consistant en le diabète, l'obésité, la colite ulcéreuse et la maladie de Crohn.

FIG. 1A

FIG. 1B

# FIG. 1C

FIG. 2A

*Parabacteroides distasonis*

FIG. 2B

FIG. 2C

*Bacteroides vulgatus*

FIG. 2D

Fitted coverage line(s) | Predicted replication origin | Literature replication origin

FIG. 2E

$R^2 = 0.98$

FIG. 2F

<5% from literature origins | Novel Origin Predictions

5-10% from literature origins | Putative errors in literature origins

>10% from literature origins

FIG. 3A
*Ruminococcus obeum*

FIG. 3B
*Eubacterium siraeum*

FIG. 3C
*Parabacteroides distasonis*

FIG. 3D
*Eubacterium eligens*

FIG. 3E
*Haemophilus parainfluenzae*

FIG. 3F
*Butyrate-producing bacterium SSC/2*

Peak-to-trough ratio
Relative Abundance

FIG. 4

FIG. 5A
Eubacterium eligens

FIG. 5B
Eubacterium rectale

FIG. 5C
Bifidobacterium adolescentis

FIG. 5D
Alistipes finegoldii

FIG. 5E
Odoribacter splanchnicus

FIG. 5F
Streptococcus parasanguinis

Predicted replication origin    Literature replication origin

FIG. 6A Alistipes shahii

FIG. 6B Ruminococcus sp. SR1/5

FIG. 6C Ruminococcus bromii

FIG. 6D Bifidobacterium longum

FIG. 6E Ruminococcus obeum

FIG. 6F Butyrate-producing bacterium SS3/4

FIG. 6G Roseburia intestinalis

FIG. 6H Coprococcus catus

FIG. 6I Faecalibacterium prausnitzii

Predicted replication origin

FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4666828 A
- US 4683202 A
- US 4801531 A
- US 5192659 A
- US 5272057 A
- US 3791932 A
- US 3839153 A
- US 3850752 A
- US 3850578 A
- US 3853987 A
- US 3867517 A
- US 3879262 A
- US 3901654 A
- US 3935074 A
- US 3984533 A
- US 3996345 A
- US 4034074 A
- US 4098876 A
- US 4879219 A
- US 5011771 A
- US 5281521 A

### Non-patent literature cited in the description

- **XU, J. et al.** *Genome Biol.,* 2012, vol. 13, R27
- **SKOVGAARD, O. et al.** *Genome Res.,* 2011, vol. 21, 1388-93
- **QIN, J. et al.** A metagenome-wide association study of gut microbiota in type 2 diabetes. *Nature,* 2012, vol. 490, 55-60
- **NIELSEN, H. B. et al.** Identification and assembly of genomes and genetic elements in complex metagenomic samples without using reference genomes. *Nat. Biotechnol.,* 2014
- **MCNEIL L K et al.** The National Microbial Pathogen Database Resource (NMPDR): a genomics platform based on subsystem annotation. *Nucleic Acids Res.,* 2007, vol. 35, D347-53
- **BAO et al.** *Journal of Human Genetics,* 28 April 2011, 1-9
- **AUSEBEL et al.** Current Protocols in Molecular Biology. Wiley, 1998
- **HAZEN et al.** Deep-sea oil plume enriches indigenous oil-degrading bacteria. *Science,* 2010, vol. 330, 204-208
- **SAMBROOK et al.** Molecular Cloning: A laboratory Manual. 1989
- Current Protocols in Molecular Biology. 1994, vol. I-III
- **AUSUBEL et al.** Current Protocols in Molecular Biology. John Wiley and Sons, 1989
- **PERBAL.** A Practical Guide to Molecular Cloning. John Wiley & Sons, 1988
- **WATSON et al.** Recombinant DNA. Scientific American Books
- Genome Analysis: A Laboratory Manual Series. Cold Spring Harbor Laboratory Press, 1998, vol. 1-4
- Cell Biology: A Laboratory Handbook. 1994, vol. I-III
- **FRESHNEY.** Culture of Animal Cells - A Manual of Basic Technique. Wiley-Liss, 1994
- Current Protocols in Immunology. 1994, vol. I-III
- Basic and Clinical Immunology. Appleton & Lange, 1994
- Selected Methods in Cellular Immunology. W. H. Freeman and Co, 1980
- Oligonucleotide Synthesis. 1984
- Nucleic Acid Hybridization. 1985
- Transcription and Translation. 1984
- Animal Cell Culture. 1986
- Immobilized Cells and Enzymes. IRL Press, 1986
- **PERBAL, B.** *A Practical Guide to Molecular Cloning,* 1984
- Methods in Enzymology. Academic Press, vol. 1-317
- PCR Protocols: A Guide To Methods And Applications. Academic Press, 1990
- **MARSHAK et al.** Strategies for Protein Purification and Characterization - A Laboratory Course Manual. CSHL Press, 1996
- **GAO, F. ; LUO, H. ; ZHANG, C.-T.** DoriC 5.0: an updated database of oriC regions in both bacterial and archaeal genomes. *Nucleic Acids Res.,* 2013, vol. 41, D90-3
- **QIN, J. et al.** A human gut microbial gene catalogue established by metagenomic sequencing. *Nature,* 2010, vol. 464, 59-65

- **RHO, M. ; TANG, H. ; YE, Y.** FragGeneScan: predicting genes in short and error-prone reads. *Nucleic Acids Res.,* 2010, vol. 38, e191
- **HUMAN, T. ; PROJECT, M.** Structure, function and diversity of the healthy human microbiome. *Nature,* 2012, vol. 486, 207-14
- **TURNBAUGH, P. J. et al.** A core gut microbiome in obese and lean twins. *Nature,* 2009, vol. 457, 480-4
- **MARKOWITZ, V. M. et al.** IMG/M-HMP: a metagenome comparative analysis system for the Human Microbiome Project. *PLoS One,* 2012, vol. 7, e40151
- **MEYER, F. et al.** The metagenomics RAST server - a public resource for the automatic phylogenetic and functional analysis of metagenomes. *BMC Bioinformatics,* 2008, vol. 9, 386
- **KARLSSON, F. H. et al.** Gut metagenome in European women with normal, impaired and diabetic glucose control. *Nature,* 2013, vol. 498, 99-103
- **SCHLOISSNIG, S. et al.** Genomic variation landscape of the human gut microbiome. *Nature,* 2013, vol. 493, 45-50
- **URBAN, A. E. et al.** High-resolution mapping of DNA copy alterations in human chromosome 22 using high-density tiling oligonucleotide arrays. *Proc. Natl. Acad. Sci. U. S. A.,* 2006, vol. 103, 4534-9
- **BAILEY, J. A. et al.** Recent segmental duplications in the human genome. *Science,* 2002, vol. 297, 1003-7
- **CHENG, Z. et al.** A genome-wide comparison of recent chimpanzee and human segmental duplications. *Nature,* 2005, vol. 437, 88-93
- **CHIANG, D. Y. et al.** High-resolution mapping of copy-number alterations with massively parallel sequencing. *Nat. Methods,* 2009, vol. 6, 99-103
- **ALKAN, C. et al.** Personalized copy number and segmental duplication maps using next-generation sequencing. *Nat. Genet.,* 2009, vol. 41, 1061-7
- **CAMPBELL, P. J. et al.** Identification of somatically acquired rearrangements in cancer using genome-wide massively parallel paired-end sequencing. *Nat. Genet.,* 2008, vol. 40, 722-9
- **MCKERNAN, K. J. et al.** Sequence and structural variation in a human genome uncovered by short-read, massively parallel ligation sequencing using two-base encoding. *Genome Res.,* 2009, vol. 19, 1527-41
- **YOON, S. ; XUAN, Z. ; MAKAROV, V. ; YE, K. ; SEBAT, J.** Sensitive and accurate detection of copy number variants using read depth of coverage. *Genome Res.,* 2009, vol. 19, 1586-92
- **MEDVEDEV, P. ; FIUME, M. ; DZAMBA, M. ; SMITH, T. ; BRUDNO, M.** Detecting copy number variation with mated short reads. *Genome Res.,* 2010, vol. 20, 1613-22
- **ABYZOV, A. ; URBAN, A. E. ; SNYDER, M. ; GERSTEIN, M.** CNVnator: an approach to discover, genotype, and characterize typical and atypical CNVs from family and population genome sequencing. *Genome Res.,* 2011, vol. 21, 974-84
- **XU, J. et al.** Genome-wide identification and characterization of replication origins by deep sequencing. *Genome Biol.,* 2012, vol. 13, R27
- **SKOVGAARD, O. ; BAK, M. ; LØBNER-OLESEN, A. ; TOMMERUP, N.** Genome-wide detection of chromosomal rearrangements, indels, and mutations in circular chromosomes by short read sequencing. *Genome Res.,* 2011, vol. 21, 1388-93
- **ALLARDET-SERVENT, A. ; MICHAUX-CHARAC-HON, S. ; JUMAS-BILAK, E. ; KARAYAN, L. ; RA-MUZ, M.** Presence of one linear and one circular chromosome in the Agrobacterium tumefaciens C58 genome. *J. Bacteriol.,* 1993, vol. 175, 7869-74
- **HINNEBUSCH, J. ; TILLY, K.** Linear plasmids and chromosomes in bacteria. *Mol. Microbiol.,* 1993, vol. 10, 917-22
- **WANG, J. D. ; LEVIN, P. A.** Metabolism, cell growth and the bacterial cell cycle. *Nat. Rev. Microbiol.,* 2009, vol. 7, 822-7
- **COOPER, S. ; HELMSTETTER, C. E.** Chromosome replication and the division cycle of Escherichia coli B/r. *J. Mol. Biol.,* 1968, vol. 31, 519-40
- **SCHAECHTER, M. ; MAALOE, O. ; KJELDGAARD, N. O.** Dependency on medium and temperature of cell size and chemical composition during balanced grown of Salmonella typhimurium. *J. Gen. Microbiol.,* 1958, vol. 19, 592-606
- **FOSSUM, S. ; CROOKE, E. ; SKARSTAD, K.** Organization of sister origins and replisomes during multifork DNA replication in Escherichia coli. *EMBO J.,* 2007, vol. 26, 4514-22
- **NIELSEN, H. J. ; YOUNGREN, B. ; HANSEN, F. G. ; AUSTIN, S.** Dynamics of Escherichia coli chromosome segregation during multifork replication. *J. Bacteriol.,* 2007, vol. 189, 8660-6
- **GAO, F. ; LUO, H. ; ZHANG, C.-T.** DoriC 5.0: an updated database of oriC regions in both bacterial and archaeal genomes. *Nucleic Acids Res.,* 2013, vol. 41, 90-3
- Human microbiome analysis. **MORGAN, X. C. ; HUT-TENHOWER, C.** PLoS Comput. Biol. 2012, vol. 8, e1002808
- **HUGHES, M. E. ; HOGENESCH, J. B. ; KOMACK-ER, K.** JTK_CYCLE: an efficient nonparametric algorithm for detecting rhythmic components in genome-scale data sets. *J. Biol. Rhythms,* 2010, vol. 25, 372-80
- **LE CHATELIER, E. et al.** Richness of human gut microbiome correlates with metabolic markers. *Nature,* 2013, vol. 500, 541-6
- **AHN, J. et al.** Human gut microbiome and risk for colorectal cancer. *J. Natl. Cancer Inst.,* 2013, vol. 105, 1907-11

- **YOSHIMOTO, S. et al.** Obesity-induced gut microbial metabolite promotes liver cancer through senescence secretome. *Nature,* 2013, vol. 499, 97-101
- **TATUSOVA, T. ; CIUFO, S. ; FEDOROV, B. ; O'NEILL, K. ; TOLSTOY, I.** RefSeq microbial genomes database: new representation and annotation strategy. *Nucleic Acids Res.,* 2014, vol. 42, 553-9
- **FLICEK, P. et al.** Ensembl 2014. *Nucleic Acids Res.,* 2014, vol. 42, 749-55
- **MARCO-SOLA, S. ; SAMMETH, M. ; GUIGÓ, R. ; RIBECA, P.** The GEM mapper: fast, accurate and versatile alignment by filtration. *Nat. Methods,* 2012, vol. 9, 1185-8
- **FRANCIS, O. E. et al.** Pathoscope: species identification and strain attribution with unassembled sequencing data. *Genome Res.,* 2013, vol. 23, 1721-9
- **NEWVILLE, M. ; INGARGIOLA, A. ; STENSITZKI, T. ; ALLEN, D. B.** *LMFIT: Non-Linear Least-Square Minimization and Curve-Fitting for Python,* 2014